Europäisches Patentamt

European Patent Office

Office européen des brevets

(19)

(11) Publication number: **0 320 213 B1**

(12) **EUROPEAN PATENT SPECIFICATION**

(45) Date of publication of patent specification: **08.03.95**  (51) Int. Cl.6: **C07C 59/305**, C07C 51/367

(21) Application number: **88311548.7**

(22) Date of filing: **06.12.88**

(54) **Improved, controlled temperature process for making 2,2'-oxodisuccinates useful as laundry detergent builders.**

(30) Priority: **10.12.87 US 130928**
**26.02.88 US 160615**

(43) Date of publication of application:
**14.06.89 Bulletin 89/24**

(45) Publication of the grant of the patent:
**08.03.95 Bulletin 95/10**

(84) Designated Contracting States:
**DE ES FR GB IT**

(56) References cited:
**EP-A- 0 236 007**

(73) Proprietor: **THE PROCTER & GAMBLE COM-
PANY
One Procter & Gamble Plaza
Cincinnati
Ohio 45202 (US)**

(72) Inventor: **Connor, Daniel S.
9217 Sagemeadow Drive
Cincinnati
Ohio 45239 (US)**
Inventor: **Kretschmar, Herbert C.
160 Julep Lane
Cincinnati**

**Ohio 45218 (US)**
Inventor: **Macbrair, Clifford Leroy, Jr.
9089 Millcliff Drive
Cincinnati
Ohio 45231 (US)**
Inventor: **Cleary, James Albert
3945 Dust Commander Drive
Hamilton
Ohio 45011 (US)**

(74) Representative: **Gibson, Tony Nicholas et al
Procter & Gamble (NTC) Limited
Whitley Road
Longbenton
Newcastle upon Tyne NE12 9TS (GB)**

Rank Xerox (UK) Business Services
(3.10/3.09/3.3.3)

**Description**

The present invention is an improved chemical process for making 2,2′-oxodisuccinate, the tetrasodium salt of which is a known laundry detergent builder. The process involves reacting maleate and malate. A novel synthesis of a suitable malate starting-material is also provided.

BACKGROUND ART

Malic acid, its salts and derivatives are well-known and useful in commerce, *inter alia* as food acidulants, and 2,2-oxodisuccinate is known as a laundry detergent builder.

Malic acid has been commercially available since the early 1960's; see Kirk-Othmer, Encyclopedia of Chemical Technology, 3rd Edition, 1981, Vol. 13, pages 103-119 and the art cited therein, all incorporated herein by reference. Kirk-Othmer refers to a patent which relates to the direct hydration of maleic acid at 180°C and 1.03-1.21 MPa (150-175 psi). A commercial synthesis of R,S-malic acid is discussed in detail by Kirk-Othmer. This synthesis involves hydration of maleic or fumaric acid at elevated temperature and pressure (180-220°C/1.4-1.8 MPa, i.e., 200 psi or above) in a titanium reactor under acidic conditions. The reaction is inefficient in that a significant amount of fumarate is generated and has to be separated from the malic acid and recycled. After a twelve-step purification process, malic acid containing less than 1% fumaric acid and less than 0.05% maleic acid is obtained. Thus, the current commercial synthesis suffers at least four serious disadvantages: first, in the inefficiency of the malic acid formation; second, high pressures are needed; third, highly corrosive acid conditions require use of a titanium reaction vessel; and fourth, elaborate and costly purification is required.

Accordingly, it will be appreciated that a simpler means for converting low-cost maleate to malate would be advantageous.

Kinetic studies on the hydration of fumarate to malate under neutral, acid and basic (sodium hydroxide) conditions have been disclosed in the art. See Rozelle and Alberty, J. Phys. Chem., Vol. 61, 1957, pages 1637-1640; Erickson and Alberty, J. Phys. Chem., Vol. 63, 1959, pages 705-709 and Bender and Connors, J. Amer. Chem. Soc., Vol. 84, 196662, pages 1980-1986. No use of multivalent metal ions is disclosed, and the studies are typically conducted in dilute aqueous solutions.

Processes for making malate and 2,2'-oxodisuccinate in metal salt form are known and disclosed in the art. Berg, U.S. Patent 3,128,287, issued April 7, 1964, states that the process of his invention comprises an efficient method of producing 2,2'-oxodisuccinic acid and malic acid and provides the following disclosure:

(Col. 1, lines 32-39)

The process of this invention involves the reaction of maleic acid with a hydroxide of calcium, barium, magnesium or strontium. In general this reaction is conducted by admixing maleic acid with an excess of the hydroxide in the presence of water. The reaction mixture is then heated for from one day to one month at temperatures ranging from 50°C to reflux temperatures.

See also Col. 1, lines 65-71, which state:

As indicated above, the process of this invention produces both malic and 2,2'-oxodisuccinic acid, the ratio of these products varies with the metal hydroxide employed in the process. When strontium and barium hydroxides are employed, an almost quantitive [sic] conversion of maleic to malic acid can be effected. However, the use of calcium and magnesium hydroxide in this process produces almost an equal mixture of malic and 2,2'-oxodisuccinic acids.

See also the examples, especially Example 1.

It is clear from the disclosure that the Berg process does not, in fact, provide a viable commercial process for converting maleic anhydride to malic acid or salts thereof; first, insofar as strontium or barium are expensive or toxic, therefore undesirable for use in making large volume food additives or laundry detergent chemicals; and second, insofar as low yields of malate are obtained using calcium or magnesium.

Accordingly, it is an object of the present invention to provide high yields of malate using calcium or magnesium.

The present invention also relates to the manufacture of 2,2′-oxodisuccinate. Regardless of the efficiency of the Berg process for producing "almost equal" mixtures of malic acid with 2,2′-oxodisuccinate, a synthesis directly leading to high yields of 2,2′-oxodisuccinate, with only low levels of malate or organic by-products such as fumarate, is not disclosed in the art. Thus, if it is desired to isolate the 2,2′-oxodisuccinate salts or formulate them into a detergent composition without automatically co-introducing substantial amounts of malate into the formulation, organic purification of the product is required. Low yield and low organic purity of the 2,2′-oxodisuccinate crude product of the Berg invention render its large-scale production commercially unattractive.

A 2,2′-oxodisuccinate synthesis process based upon Berg is also disclosed by Lamberti et al, U.S. Patent 3,635,830 issued January 18, 1972. The Lamberti et al process shares the disadvantages of the Berg process.

Lamberti et al further discloses that 2,2′-oxodisuccinate salts are useful laundry detergent builders.

Matzner et al, Tenside Detergents, 1973, Vol. 10, 239-245, writing in the context of synthesis of 2,2′-oxodisuccinates as detergent builders, state, "A more economical process would have to be developed to make this a practical (builder) candidate."

Notwithstanding the more recent disclosure of Nieuwenhuizen et al, J. Amer. Oil Chemists' Soc., Vol. 60, 1983, pages 44-48, that it is possible to conduct a laboratory synthesis of 2,2′-oxodisuccinates by addition of (preformed) malic acid to maleic acid in aqueous alkaline medium in the presence of a divalent cation, preferably Ca2+, no commercially viable industrial process for making 2,2'- oxodisuccinates appears to have been disclosed in the art.

The Berg and Lamberti et al patents cited above are incorporated herein by reference, especially insofar as they also include disclosure of conversion of crude calcium or magnesium containing product of the above-cited processes to 2,2'-oxodisuccinate monovalent cation salts (e.g., sodium salts). As noted, separation of 2,2'-oxodisuccinate from other organic species such as malate is also disclosed.

The formation of 2,2'-oxodisuccinate by means of a two steps process is also known from the disclosure of EP-A-236007 which teaches:

i)- Formation of 2,2'-oxodisuccinate by combining one maleate reactant and one malate reactant at a temperature below 120°C and maintaining this reaction mixture at a temperature of at least 60°C, sufficient to permit ether-bond formation between these two reactants.

ii)-Arrest of reaction by cooling the reaction mixture to less than about 50°C

However, there still remains a clear need for an improved 2,2'-oxodisuccinate-forming process. A high-yield process, which is capable of improving the economics and/or practicality of 2,2'-oxodisuccinate production, would be especially useful to the user and manufacturer of detergent compositions. A cost-effective, large-scale process should very preferably minimize the need for organic purification of the crude 2,2'-oxodisuccinate product.

It is an object of the present invention to provide such an improved 2,2'-oxodisuccinate synthesis process, wherein the ether-bond forming reaction:

maleate + malate → 2,2'-oxodisuccinate

is carried out efficiently and in high yield.

It is further object of the invention to provide a process which is usable in an overall conversion of maleic anhydride to 2,2'-oxodisuccinic acid or sodium salts thereof. Yet another object of the invention is the provision of a process for cost-effectively preparing laundry-detergent grade 2,2'-oxodisuccinate salts on a large scale, wherein organic purification is optional rather than essential.

## SUMMARY OF THE INVENTION

The instant process encompasses chemical conversion of a particularly defined organic component to 2,2'-oxodisuccinate. Yields of 2,2'-oxodisuccinate are generally at least 80%, based on the weight of the organic component.

Whatever the precise chemical forms of the starting-materials, the 2,2′-oxodisuccinate-making process is effective when the starting-materials comprise:

I. an organic component comprising maleate and preformed malate;

II. a divalent metal cation component selected from calcium, magnesium and mixtures thereof;

III. an alkali component selected from hydroxide and hydroxide-forming (for example, oxide) anions;

and the following particularly important additional component:

IV. a solubilizing monovalent cation component selected from sodium, potassium and mixtures thereof.

For best results, indeed essentially when it is desired to secure 2,2′-oxodisuccinate yields of 85% and higher, these components are used at the particular levels given in detail hereinafter. Levels and proportions at the start of the process are specified on a fully anhydride-hydrolyzed and neutralized basis and on the basis that no conversion of the organic component to 2,2′-oxodisuccinate has occurred. No components other than water and I-IV are essential in the 2,2′-oxodisuccinate-making process.

The chemist will recognize that various chemical forms of conventional starting-materials can be used. For example, a starting-material mixture made from maleic anhydride, water, D,L-malic acid, calcium hydroxide and sodium hydroxide is suitable for use herein. Another suitable starting-material mixture can be made by conventionally reacting maleic acid and calcium carbonate in water, evolving carbon dioxide and forming calcium maleate; then admixing D,L-malic acid and sodium hydroxide.

Aqueous concentration is important in the present process. The process is generally not carried out in highly dilute aqueous media. Use of concentrated aqueous media, provided that these are not unworkably viscous, is highly preferred. The water content herein is specified on a fully hydrolyzed and neutralized basis; i.e., water removed, for example in hydrolyzing maleic anhydride to maleate, and water formed, for example in neutralizing acids and bases, are taken into account in specifying the weight percentages of water. Some evaporation losses may occur during the process; preferably, such losses are minimized by using covers or condensers on the process equipment. Alternatively, water losses can be compensated for, by adding evaporation-compensating amounts of water at any stage during the process. In any event, evaporation in the present 2,2'-oxodisuccinate-making process is not permitted to the extent of forming unworkably viscous mixtures. It is in this sense that the term "fluid" is used hereinafter in describing mixtures of the starting-materials or crude product mixtures.

As noted, the above-specified component IV is particularly important in the instant 2,2'-oxodisuccinate-making process. The specified water-soluble sodium cations, potassium cations or mixtures thereof are conventional materials. Used at levels of at least 1% by weight of the starting-material mixture, their effect in the process is a significant, albeit not necessarily perfect solubilization of aqueous mixtures of components I-IV. Component IV levels in the range from 3% to 20% are preferred. Levels in the range from 3% to 16% are especially preferred. Without being bound by mechanistic theory, and irrespective of whether solubilization is fully and directly responsible for the results obtained, 2,2'-oxodisuccinate yields in excess of 80% cannot be obtained in the absence of this component.

The present 2,2'-oxodisuccinate-making process generally involves reacting the mixed starting-materials in water for a total duration of reaction of at least 12 hours, at temperatures of 110°C or lower (preferably in the range of 20°C to 110°C). Pressures are not critical. The total duration of reaction can be long, e.g., 400 days.

It has been discovered that in order to produce 2,2'-oxodisuccinate at yields of 80% and higher, it is necessary to react fluid, aqueous alkaline mixtures of the starting-materials without overly extended exposure to high temperatures. Thus, in the instant process, the total time at any temperature above 100°C does not exceed 1.5 hours and the total time at any temperature above 90°C does not exceed 4.5 hours and the total time at any temperature above 80°C does not exceed 13.5 hours and the total time at any temperature above 70°C does not exceed 1.7 days and the total time at any temperature above 60°C does not exceed 5.1 days.

It has also been found that overly extended exposure to relatively lower temperatures is also to be avoided in order to secure 2,2'-oxodisuccinate yields of 80% and higher. Thus, in the instant process, the total time at any temperature above 50°C does not exceed 15 days and the total time at any temperature above 40°C does not exceed 46 days and the total time at any temperature above 30°C does not exceed 137 days.

When these provisions are respected, the present process reproducibly provides the desired yields of 2,2'-oxodisuccinate.

It will be appreciated that the best reaction temperatures and times will vary to some extent in dependence of the precise levels of components I-IV and in dependence of the aqueous concentration at which the process is carried out. More narrowly defined temperature-time provisions are also given hereinafter for processes according to the invention in which magnesium and potassium are used to only limited extent, as well as for processes in which only calcium and sodium cations are present by way of components II and IV respectively.

The process of the invention cannot be continued for indefinitely long periods, since 2,2'-oxodisuccinate yields reach maximum (depending on the reaction temperatures) and then tend to fall. Thus, the crude product mixtures are subjected to an organic reaction-arresting and workup procedure, which typically involves diluting the crude product mixture formed in the process and precipitating calcium carbonate using warm aqueous $Na_2CO_3/NaHCO_3$.

It is generally preferred to avoid using high temperatures, excepting for the early stages of the process. In other terms, it is preferred to avoid reheating, to high temperatures for significant periods, any crude product mixtures which have already been reacted to reach high 2,2'-oxodisuccinate yields. Thus, the preferred embodiments of the invention include isothermal processes of duration greater than 12 hours, for example at an approximately steady temperature in the range 40°C-68°C, as well as non-isothermal processes associated with specific temperatures and times. For the highest 2,2'-oxodisuccinate yields (typically at least 85%-90%), non-isothermal processes are generally preferred. Especially preferred on the basis of yield are narrowly defined non-isothermal embodiments, having (a) an elevated temperature primary reaction procedure and (b) a lower temperature maturation procedure. In these embodiments, (a) is typically a relatively short, relatively warm reaction while (b) is a relatively longer reaction carried out at relatively lower temperatures (preferably, lower than step (a) by 10°C or more).

If yields in the range 80%-85% are acceptable, and it is desired to carry out the process relatively rapidly, the practitioner may use either isothermal or non-isothermal embodiments of the invention, such as those illustrated in detail hereinafter, provided that total reaction times are always in excess of 12 hours.

An advantage of the instant process is that only the elevated temperature primary reaction (a) need be carried out in a relatively expensive stirred reactor. The maturation procedure (b) typically requires no more than an unstirred, preferably thermally insulated, holding-tank, or even a simple tank car.

Even more significantly, the invention for the first time provides direct access to a large-scale commercial production of 2,2'-oxodisuccinate sodium salts for use as builders, for example in laundry detergents and other consumer products.

The present invention also encompasses an improved aqueous process for converting a maleate feedstock to malate in very high yield. The product is typically an unusual granular form of calcium malate, magnesium malate or mixtures thereof, having particularly good odor and color. This material can be used as a source of malate for use in the above-summarized 2,2'oxodisuccinate making process. The malate-making process requires use of temperatures above reflux and therefore generally uses a sealed or mildly pressurized reaction vessel. High pressures are unnecessary and the reaction does not involve formation of a corrosive, acidic reaction mixture. Insofar as the product salts may simply be acidified to liberate malic acid, the invention also encompasses an improved synthesis of malate in acid form. Used as a starting-material in the above-summarized 2,2'-oxodisuccinate-making process, the product of the improved malate synthesis is exceptionally useful for making 2,2'-oxodisuccinate laundry detergent builder salts, but may well also be of great utility to the manufacturer of foods and other consumer products.

## DETAILED DESCRIPTION OF THE INVENTION

### Malate-Making

The instant invention encompasses a process for making malate by at least one step of maintaining, in a reaction vessel (preferably sealed) at a temperature of 120°C or above (more preferably, 130°C to 220°C, most preferably 140°C to 190°C) for a time of at least 10 minutes (more preferably 10 minutes to 16 hours, most preferably 30 minutes to 6 hours), an aqueous reaction mixture comprising:

I. maleate, or mixtures thereof, with one or more of 2,2'-oxodisuccinate, fumarate and malate;

II. divalent cations selected from calcium, magnesium and mixtures thereof; and

III. sufficient hydroxide excess to render said reaction mixture alkaline;

whereby said malate is secured in yields up to and exceeding 90%, (more preferably, exceeding about 99%).

The product may readily be isolated as a solid salt form of said divalent cation component II.

A preferred embodiment of the invention uses, for malate-making, a reaction mixture consisting essentially of the above-defined components I, II and III and water.

In another preferred embodiment, for malate-making, said reaction mixture comprises said components and is substantially free from sodium or potassium cations (it is, however, possible to have such cations present in limited amounts).

In the simplest embodiment of the malate-making process, component I is used in the form of maleic anhydride or maleic acid, and components II and III can simultaneously be provided in the form of calcium hydroxide. As illustrated in this embodiment, the malate-making process has highly preferred ranges of composition of the reaction mixture when particular calcium/maleate mole ratios and particular aqueous concentrations are used. Thus, for malate-making, the calcium/maleate mole ratio is generally greater than 1:1. Typically, the calcium/maleate mole ratio is at least 1.1:1, more preferably greater than 1.3:1 and less than 1.7:1, most preferably from 1.45:1 to 1.55:1. It will be appreciated that in this embodiment of the malate-making process, the hydroxide excess (component III) is inherently fixed by use of calcium hydroxide as the source of both divalent metal and base. More generally it will be seen that, for malate-making, the components II and III can separately be selected; for example, if calcium carbonate is used in a preneutralizing step (wherein calcium carbonate and said component I are mixed and carbon dioxide is evolved prior to sealing said reactor or prior to carrying out the malate-forming process of the invention) and the reaction mixture is then formed using calcium hydroxide, amounts of components II and III are independently controllable.

Particularly preferred component II/component I, (i.e., calcium/maleate) mole ratios being as specified above, preferred mole ratios of excess hydroxide/maleate (i.e., component III/I) herein are generally at least 0.2:1, more preferably from 0.6:1 to 1.4:1, most preferably from 0.90:1 to 1.1:1. As noted, there also exist preferred aqueous concentrations, or water contents, of the reaction mixture. In the malate-making process of the invention, the water content is generally in the range from 10% to 95 wt%, more preferably, 40% to 90%, most preferably, 45% to 73 wt% of the reaction mixture.

Very surprisingly, in the preferred range of calcium/malate mole ratios (and especially in the most preferred range, 1.45:1 to 1.55:1), the reaction mixtures become more easily workable, forming an aqueous dispersion or suspension which comprises a unique, granular malate reaction product wherein the excess calcium hydroxide is intimately comixed, and wherein malate is formed in best yields. This product is unique and crystallographically distinct from common calcium malate, or calcium hydroxide, or simple mixtures of the two. Likewise, the product is crystallographically distinct from that prepared according to Example 1 of the above-cited Berg patent.

In its composition aspects, the invention encompasses these unusual malate-containing solid products, another very useful form of which can be obtained by using mixed calcium and magnesium cations at calcium:magnesium mole ratios in the above-summarized process of from 0.99:0.01 to 0.01:0.99, more preferably, from 0.98:0.02 to 0.80:0.20, most preferably, from 0.97:0.03 to 0.90:0.10.

The products of the malate-making process can directly be used for making malic acid. Thus, by using the above-described process step and treating the product thereof with mineral acid or any other convenient source of exchangeable $H^+$, malic acid is secured.

By using the identical malate-making process and treating the product thereof with an additional amount of the component I (the reaction mixture now differing in composition in that both malate and maleate are essentially present) in the presence of solubilizing cation (such as sodium or potassium), 2,2'-oxodisuccinate is formed in high yield, as is further described in detail hereinafter. It is a remarkable feature of the present invention that by conducting a two-step 2,2'-oxodisuccinate synthesis process, using the first step of forming malate salts in solid form near-quantitatively (at relatively high temperatures), and using another step of reacting the malate salts with additional maleate and solubilizing amounts of monovalent cation (at relatively lower temperatures), a more efficient conversion of component I to 2,2'-oxodisuccinate can be secured.

The invention has other preferred embodiments which are described in detail herein. Thus, in a typical commercial process for malate-making involving recycle steps, component I uses a mixture of maleate with one or more of 2,2'-oxodisuccinate, malate or fumarate. Indeed, it has been discovered that the malate-making process is generally capable of interconverting a mixture of these organic moieties to form malate in high yield. Component I will have a composition (weight %) in the following ranges for best results:

| Weight % | Generally Acceptable | Preferred | Most Preferred |
|---|---|---|---|
| maleate | 20-100 | 50-100 | 90-100 |
| malate | 0-50 | 0-30 | 0-10 |
| 2,2'-oxodisuccinate | 0-50 | 0-30 | 0-10 |
| fumarate | 0-50 | 0-30 | 0-10 |

When component I has the above range of composition, the critical composition ratios illustrated in the simple embodiment above naturally require a more complex definition: the ratios will be calculated based on maleate feedstock taken as a whole, rather than on maleate alone. Making proper allowance for the total molar amount of carboxylate used as maleate, malate, 2,2'-oxodisuccinate and fumarate, the ratios in general are still in accordance with the simple illustration given above.

The malate-making process of the instant invention is a high-yield process. Yields of 90% by weight or more are the norm. (Yields herein are based on weight percentage of the organic feedstock, i.e., on the total weight of maleate + malate + 2,2'-oxodisuccinate + fumarate used or present in the reaction mixture. See the analytical protocol hereinafter for further detail). When net yield is the only consideration, yields of 99% or more can readily be achieved using the invention.

6

Maximizing yield of malate, however, is not the only consideration herein. The invention also provides selections of reaction conditions and compositions of the malate-making reaction mixtures especially suited for obtaining the malate-containing product herein as an unusual, easily handled granular solid. Thus, by selecting a reaction vessel having conventional means to drain spent liquors (e.g., by gravity), and by using specific, generally high, component II/I and component III/I mole ratios as further discussed hereinafter, the liquid phase of the reaction mixture may simply be drained at the end of the malate-making step, leaving the granular product in the reactor. This facilitates subsequent processes (e.g., malic acid or 2,2′-oxodisuccinate preparation) in the same reactor as is used for the malate synthesis.

Alternatively, separate reactors may be used for making each of the malate salts, malic acid and 2,2′-oxodisuccinate as provided for herein. For example, by modifying the reaction conditions and composition of the malate-making reaction mixture somewhat, a finer form of the above-mentioned solid granular malate-containing product is secured as a pumpable slurry, which can be readily transferred into the separate reactors used for converting the malate product of the invention to malic acid or 2,2′-oxodisuccinate. Such modification of the process uses the preferred temperatures and reaction times at the high end of the indicated ranges, or uses component I containing specific, limited amounts of fumarate, or uses component II containing specific, limited amounts of magnesium, to secure the desired slurries. These and other considerations are more fully described hereinafter.

A further aspect of the invention of considerable importance is that of the discovery of a process capable of controlling fumarate levels in product malate, or in product malic acid or 2,2′-oxodisuccinate derived therefrom.

The results herein are especially surprising in view of the facts on one hand that Berg, despite use of varying reaction conditions, reports only mixtures of malate and 2,2′-oxodisuccinate. In our hands, the Berg process, in fact, also forms fumarate. The Berg process using calcium or magnesium is consistent with base-catalyzed Michael addition of maleate to $in-situ$ formed malate, and fumarate is presumably derived, at least in part, from decomposition of 2,2′-oxodisuccinate. The present process for making malate avoids or minimizes such side-reactions, thereby selectively maximizing malate formation. On the other hand, current commercial processes for manufacture of malic acid, while operating at high (above reflux) temperatures similar to those herein, are also subject to fumarate-by-product formation. Without being bound by theory, the instant process, through formation of an unusual (typically alkaline, granular), solid form of malate as the divalent metal salt, may kinetically stabilize and protect the reaction mixture against the fumarate-forming tendency. It appears that a total of four organic species, i.e., maleate, malate, fumarate and 2,2′-oxodisuccinate, are involved in the instant reaction. Irrespective of the mechanisms involved, the present invention secures the means to control the interconversion of these species, thereby resulting in high yields of malate.

The malate-making process of the invention is not believed to have any true pressure criticality. However, since it generally involves heating an aqueous mixture at temperatures above reflux, the malate-making reaction is usually carried out in a sealed reaction vessel of conventional construction, such as 316 SS (stainless steel). This reaction vessel need not be of titanium, nor need it be capable of withstanding high pressures, since the process (other than in handling of raw materials) is not corrosive and operates satisfactorily at low pressures. Pressures as low as 30 psig (0.21 MPa) can be used; more typically, the process operates at pressures in the range up to 150 psig (1.03 MPa). Host preferably, pressures from 35 to 85 psig (0.24 to 0.59 MPa) are used. If desired, it is possible to operate at higher pressures but to no additional advantage. It is also possible to conduct the malate-making reaction in a reaction vessel which is not "sealed" in the conventional sense of the term; for example, evaporation of water is allowable provided that the reaction mixture does not boil dry at the reaction temperatures used. For this purpose, provision of a pressure head in the above-indicated range, or higher, maintained in the reaction vessel for the indicated reaction times, is sufficient. Thus, the reaction vessel may make use of pressurized, superheated steam introduced by means of direct injection, and may be equipped with steam outlets and/or pressure relief valves. Other aspects of reactor design, e.g., stirring and heating means, are conventional.

Reaction temperatures for the malate-making process herein are above reflux, generally at or above 120°C. More preferably, temperatures in the range 130°C to 220°C are used. Most preferably, temperatures are in the range 140°C to 190°C.

Reaction times for the malate-making process are of at least 10 minutes, more preferably 10 minutes to 16 hours, most preferably 30 minutes to 6 hours. Reaction time is generally measured as of completion of loading of all the components of the malate-forming reaction mixtures into the reactor and bringing the mixture as rapidly as possible to the reaction temperature. Naturally, it will be appreciated that shorter reaction times are preferably accompanied by selection of higher reaction temperatures within the indicated ranges.

Components of Malate-forming Reaction Mixture

The components herein are component I, (having the above-tabulated composition), components II (calcium, magnesium or mixtures of these divalent cations), and III excess base (i.e., hydroxide ion); as noted, water is also present. As noted in a simple illustration of a preferred process according to the invention summarized above, component I may be in organic acid form and both components II and III together can conveniently be provided simultaneously, for example, as calcium hydroxide. Alternatively, It is possible to adjust the amounts of each of components I, II and III independently.

In general, the malate-making process uses more than one mole of component II for each mole of maleate feedstock I. When the maleate feedstock comprises only dianions, e.g., when comprised of maleate and fumarate or maleate, fumarate and recycled malate, the preferred component II/I mole ratios are as given in the simple illustration of the process (using maleic anhydride and calcium hydroxide) hereinabove. When tetraanions, i.e., 2,2'-oxodisuccinate, are also present in the maleate feedstock, the convention is used, for purposes of calculating reactant amounts, of expressing the total molar amount of component I on a divalent anion basis. In general, moles (I) = moles dianions in the sum of the moles of maleate + malate + 2,2'-oxodisuccinate + fumarate charged in the malate-making reaction. Each mole of 2.2'-oxodisuccinate contributes 2 moles of dianion equivalents.

In the malate-making process herein, water is invariably present at least in an amount sufficient to allow reaction to occur (10 wt%). In general, the malate-forming reaction mixture comprises 10% to 95%, more preferably 40% to 90%, most preferably 45% to 73% water. Also essential is that the reaction mixture should be alkaline, i.e., in combining I, II and III in the presence of water, the reaction mixture should be neutralized and, further, be rendered alkaline to the extent determined by amount of the excess base component, III. The moles of $H^+$ and $^-OH$ involved in the neutralization of the reaction mixture (these amounts inherently resulting from the chemical form of the reactants) naturally form water, and are accounted for in defining the reaction mixture as aqueous. further, in stating the water content of the reaction mixtures herein, water formed by neutralization is taken into account.

In general, the malate-making process herein uses a mole ratio of components III:I (i.e., excess base:organic component) of at least 0.2:1. Preferably, this ratio is in the range from 0.6:1 to 1.4:1, most preferably, from 1.45:1 to 1.55:1.

The organic composition of the type I component being as noted, one satisfactory malate-making process according to the invention makes use of a type I component consisting essentially of from 20% to 100% maleate, from 0% to 50% fumarate, from 0% to 50% 2,2'-oxodisuccinate and from 0% to 50% malate. More highly preferred is to use a high proportion of maleate, together with only limited amounts of one or more of the remaining three organic moieties malate, 2,2'-oxodisuccinate and fumarate. Thus, as noted, a more preferred feedstock I comprises 50-100% (most preferably 90-100%) maleate, the balance of I being malate, 2,2'-oxodisuccinate, fumarate or mixtures thereof. Typically, most highly preferred commercial processes based on the invention accommodate recycled (or initially charged) malate, 2,2'-oxodisuccinate or fumarate amounts each in the range of from 0% to 10% by weight of the total of component I.

The malate-making process of this invention is effective where magnesium alone is used as component II. However, if a single divalent cation is used, calcium should be used as component II to secure the highest yield. Remarkably, if component II consists essentially of mixtures of calcium with relatively small proportions of magnesium, malate yields of greater than 99% are secured. When using calcium/magnesium mixtures as component II herein, the calcium/magnesium mole ratios are typically from 0.99:0.01 to 0.01:0.99, more preferably, from 0.98:0.02 to 0.80:0.20, most preferably, from 0.97:0.03 to 0.90:0.10.

Generally, the malate-making process of the invention is operated using aqueous alkaline reaction mixtures consisting essentially of components I, II, III and water. However, other cations, such as monovalent metal cations, may additionally be present. When component II consists essentially of calcium, it has been discovered that the total amount of additional, soluble monovalent cations should be controlled at or below 0.4 mole fraction of the total of non-hydrogen cations in order to achieve optimum yields. In general, expressing the total amount of non-hydrogen (monovalent and polyvalent) cations in the malate-forming reaction mixture as 1 mole (1.0 mole fraction), one preferred process of the invention uses a total non-hydrogen cation composition comprising or consisting of from 0.8 to 1.0 (more preferably 0.9 to 1.0, most preferably 1.0) mole fraction calcium, magnesium or mixture thereof. Optionally, in this preferred process, sodium, potassium, mixture of said sodium and potassium, or equivalent solubilizing monovalent cation, e.g., tetraalkylammonium or the like, can be present at levels of from 0 to 0.2, e.g., 0.1 mole fraction of the total of non-hydrogen cations.

8

## Chemical and Physical Forms of Reactants

In general, the malate-producing and subsequent malic acid or 2,2'-oxodisuccinate-making process steps herein are not particularly limited with respect to the chemical or physical form of the reactants, provided that reaction mixtures having the specified proportions of the essential components are used. An extensive list of useful reactants can be drawn up; this list includes any of the maleates, malates, 2,2'-oxodisuccinate, fumarates, oxides, hydroxides, carbonates and bicarbonates of calcium or magnesium. Likewise included are maleic anhydride (preferred), maleic acid, fumaric acid and 2,2'-oxodisuccinate acid as well as any mixed or partial salt of the foregoing cations and anions.

As noted, sodium, potassium and similar solubilizing monovalent cations in water-soluble salt form are optionally used and are only tolerated in the malate-making process at limited levels, contrasting with the 2,2'-oxodisuccinate-making process discussed hereinafter wherein the solubilizing effect of such cations is essential. Since the chemical forms of such monovalent cations include tetrasodium 2,2'-oxodisuccinate and mixed sodium/calcium 2,2'-oxodisuccinate from a 2,2'-oxodisuccinate-making step (described hereinafter) into the malate-making step should preferably be limited in accordance with the above-noted limitations on composition of component II and on overall non-hydrogen monovalent cation content of the malate-making reaction mixtures.

In general, the chemical form of the non-hydrogen cations herein will be that of oxide, hydroxide such as calcium hydroxide, magnesium hydroxide or the like, or inert anion salts. For purposes of preneutralizing component I, salts such as calcium carbonate may be used. Inert anions, e.g., sulfate, can optionally be present herein; but, most preferably, inert anions are not used. Process aids, e.g., silicates, aluminates, surfactants and hydrotropes, can optionally be used in the processes of the invention. Low levels of fumarate or magnesium are desirable in the starting compositions of the malate-making process herein (preferred malate, malic acid or 2,2'-oxodisuccinate product compositions, however, typically have fumarate levels below 7% by weight of the organic moieties).

## Embodiments of the Invention Maximizing Yields of Malate

Maximum yields of malate in the malate-forming process of the invention are readily obtainable by simply selecting temperatures, times, proportions and chemical forms of components I, II, III, and amounts of water, each as indicated hereinabove.

It will be appreciated that, for best results, the above-mentioned parameters will not be selected independently from one another. Thus, use of the lowest reaction temperatures is generally accompanied by selection of longer reaction times, and *vice-versa*. See, for example, Table 1 and the tabulated results in Examples II-XV hereinafter; in Example II, a yield of 88.8% is obtained by selecting a reaction temperature of 120°C and the rather long reaction time of 16 hours. In contrast, and more preferably, at the higher temperature (165°C) of Example XIV, a yield of 94.9% is obtained after only one hour.

Table 1 provides detailed nonlimiting illustrations of conditions under which best possible yields of malate may be obtained. (See the examples, especially Examples I, XV, and XX hereinafter, for additional procedural details.)

9

## TABLE I

| Embodiment No. | Component I Moles (Wt. %) | Component II Moles (Ca²⁺/Mg²⁺) | Component III (⁻OH excess, Moles) | Water Content (Wt. %) |
|---|---|---|---|---|
| 1 | maleate 1 (100%) | Ca²⁺ 1.33 Mg²⁺ 0.17 | 1.0 | 70.6 |
| 2 | maleate 1 (100%) | Ca²˜ 1.45 Mg²⁺ 0.05 | 1.0 | 70.3 |
| 3 | maleate 1 (100%) | Ca²⁺ 1.45 Mg²⁺ 0.05 | 1.0 | 70.3 |
| 4 | maleate 1 (100%) | Ca²⁺ 1.455 Mg²⁺ 0.045 | 1.0 | 70.3 |
| 5 | maleate 1 (100%) | Ca²⁺ 1.40 Mg²⁺ 0.00 | 0.8 | 71.3 |

| | | | | |
|---|---|---|---|---|
| 6 | maleate 1 (100%) | Ca²⁺ 1.5 Mg²⁺ 0.00 | 1.0 | 70.2 |
| 7 | maleate 1 (91%) maleate 0.1 (9%) | Ca²⁺ 1.50 Mg²⁺ 0.00 | 0.9 | 68.9 |
| 8 | maleate 1 (100%) | Ca²⁺ 1.20 Mg²⁺ 0.00 | 0.4 | 52.7 |
| 9 | maleate 1 (100%) | Ca²⁺ 1.30 Mg²⁺ 0.00 | 0.6 | 72.4 |

TABLE I (continued)

| Embodiment No | Moles II/I | Moles III/I | Composition of Component II (Ca:Mg Mole Ratio) | Chemical Form of Reactants/Order Charged |
|---|---|---|---|---|
| 1 | 1.5:1 | 1:1 | 0.89:0.11 | Footnote 1 |
| 2 | 1.5:1 | 1:1 | 0.97:0.03 | Footnote 2 |
| 3 | 1.5:1 | 1:1 | 0.97:0.03 | Footnote 3 |
| 4 | 1.5:1 | 1:1 | 0.97:0.03 | Footnote 4 |
| 5 | 1.4:1 | 0.8:1 | 1.00:0 | Footnote 5 |
| 6 | 1.5:1 | 1:1 | 1.00:0 | Footnote 6 |
| 7 | 1.5:1 | 0.9:1 | 1.00:0 | Footnote 7 |
| 8 | 1.2:1 | 0.4:1 | 1.00:0 | Footnote 8 |
| 9 | 1.3:1 | 0.6:1 | 1.00:0 | Footnote 9 |

TABLE I (continued)

| Embodiment No. | Reaction Temperature °C | Reaction Time Hrs. | Wt% (HPLC) Malate | Wt% (HPLC) 2,2"-oxo-disuccinate |
|---|---|---|---|---|
| 1 | 180 | 6 | 98.0 | 1.3 |
| 2 | 145 | 6 | 98.9 | 0.3 |
| 3 | 165 | 6 | 99.2 | 0.2 |
| 4 | 148 | 7 | 98.8 | 0.3 |
| 5 | 165 | 6 | 98.1 | 0.7 |
| 6 | 180 | 2 | 96.0 | 2.4 |
| 7 | 165 | 7 | 99.2 | 0.2 |
| 8 | 180 | 6 | 95.8 | 1.8 |
| 9 | 145 | 6 | 95.0 | 2.1 |

TABLE I (continued)

| Embodiment No. | Wt% (HPLC) Fumarate | Wt% (HPLC) Maleate | Product Character |
|---|---|---|---|
| 1 | 0.5 | 0.2 | Pumpable slurry of granular malate |
| 2 | 0.8 | -0- | Pumpable slurry of granular malate |
| 3 | 0.6 | -0- | Pumpable slurry of granular malate (See Example XX) |
| 4 | 0.9 | -0- | Pumpable slurry of granular malate |
| 5 | 1.1 | -0- | Pumpable slurry of granular malate |
| 6 | 1.6 | -0- | Coarse granules readily separable separable by decantation or infiltration from the aqueous liquor (See Examples XV, XVI) |
| 7 | 0.6 | -0- | Coarse granules |
| 8 | 2.4 | -0- | Hard soild mass |
| 9 | 2.9 | -0- | Hard solid mass |

TABLE I (Footnotes)

[1] 1 mole maleic anhydride; 433.7g water*; 0.17 mole magnesium hydroxide and 1.33 moles calcium hydroxide.

[2] 1 mole maleic anhydride 431.7g water*; 0.05 mole magnesium hydroxide and 1.45 moles clacium hydroxide.

[3] 1 mole maleic anhydride 431.7g water*; 0.05 mole magnesium hydroxide and 1.45 moles calcium hydroxide.

[4] 1 mole maleic anhydride 431.7g water*; 0.045 mole magnesium hydroxide and 1.055 moles calcium hydroxide.

[5] 1 mole maleic anhydride 438.3g water*; 1.4 moles calcium hydroxide.

[6] 1 mole maleic anhydride 431.0g water*; 1.5 moles calcium hydroxide.

[7] 1 mole maleic anhydride 441.0g water*; 1.4 moles calcium hydroxide; 0.1 mole calcium malate.

[8] 1 mole maleic anhydride 180.0g water*; 1.2 moles calcium hydroxide.

[9] 1 mole maleic anhydride 463.7g water*; 1.3 moles calcium hydroxide.

*Total water including that liberated in neutralization.

As noted hereinabove, absolute maximization of yield is not the only consideration herein, and Table 1 also illustrates especially preferred embodiments of the invention where it is desired to isolate the granular solid product (e.g., by filtration; see embodiment 6 in the table) or where it is desired to obtain a readily pumpable slurry of the same granular form of malate (having a lower mean particle size; see embodiments 1-5).

Table 1 also illustrates high-yield malate synthesis where the reaction mixture forms a less desirable hard mass (see embodiments 8 and 9), which lacks the attractiveness of handling displayed by the coarse or fine (pumpable) granular forms. The latter, more desirable, forms of the product arise at component II/I mole ratios within the preferred (>1.3:1 and ≤1.7:1) ranges, especially good results being obtained at II/I mole ratios of 1.4:1 or 1.5:1. In contrast, using a II/I mole ratio of 1.3:1 or less (see embodiments 8 and 9), the less desirable hard mass of malate product is obtained. For purposes of comparison, these less desirable II/I mole ratios apparently tend to correspond with those used by Berg and Lamberti et al, cited hereinabove.

In connection with the preferred embodiments of the malate-making invention, it is also generally advantageous to use relatively high masses of reactants in relation to amounts of water. Note that the terms "aqueous concentration" and "pH" are generally avoided herein, in view of the fact that the malate-forming process step is a two-phase reaction, so that pH does not accurately reflect the amount of hydroxide excess in the reaction mixture. The term "aqueous concentration" is likewise unuseful, in view of the generally high proportions of reactant species which are not in solution at any given point in time.

Also, in connection with Table 1, it is noteworthy that the combined advantages of high yield, high purity of the organic component of the product, and readily pumpable product all occur simultaneously at the illustrated calcium:magnesium molar proportions (see embodiments 1-4). Likewise beneficial to improve formation of a fine, pumpable granular product are any of: high reaction temperatures, longer reaction times, or presence of limited amounts of malate or fumarate in component I (see also, for example, embodiment 7, the product of which is also granular, or Examples XXIII or XXVI hereinafter).

Preferred Embodiments of the Malate-Making Process Involving Particular Selections of Reactant Forms or Involving Recycle

The malate-making process herein may make use of particular selections of reactant forms, or may involve recycle, depending upon economical sources or recycle streams available to the manufacturing facility. Most simply, as illustrated hereinabove, maleic anhydride and calcium hydroxide will suffice.

However, the invention also encompasses a malate-making process using recycle of a portion of said component I; in a simple illustration, fresh maleate (0.8 mole fraction) and recycled fumarate (0.2 mole fraction) is charged in the malate-making reactor together with the components II, III and water as specified above. In more complex illustration using a preneutralization step involving calcium carbonate (which may optionally be a recycled calcium carbonate stream), the invention encompasses an aqueous alkaline process for securing malate in a solid, alkaline, calcium or mixed calcium/magnesium salt form by the steps comprising, in sequence:

1. reacting maleic anhydride or maleic acid and calcium carbonate to form a mixture;
2. permitting carbon dioxide to evolve;
3. rendering said mixture alkaline using calcium hydroxide or mixture thereof with magnesium hydroxide; all provided that said reactants are used at a (calcium + magnesium):maleate molar ratio in the range from greater than 1.3:1 to less than (or equal to) 1.7:1, a calcium:magnesium mole ratio in the range from 1.00:0.00 to 0.80:0.20, a water content in the range from 45% to 73% and an excess of hydroxide of from 0.6 to 1.4 moles per mole of maleate used; and
4. reacting said mixture in a sealed vessel at temperatures in the range from 140°C to 190°C for a period of from 0.5 to 6 hours.

In this illustration, it is readily possible to use a fumarate-containing maleate reactant in said step 1 to the extent of not more than 0.2 moles of fumarate being present per mole of said maleic anhydride or maleic acid added in each cycle.

Another embodiment of the invention, highly preferred when the malate-making step is coupled with a subsequent 2,2'-oxodisuccinate-formingstep (see the disclosures hereinafter), uses the following reactants to charge the malate-making reactor:water, calcium carbonate filter cake from the 2,2'-oxodisuccinate process (said filter cake containing limited amounts of sodium 2,2'-oxodisuccinate salts) and maleic anhydride. These reactants are charged in any order, and carbon dioxide evolution is allowed to reach completion. Finally, excess hydroxide (component III) and the balance of the necessary calcium are added simultaneously as calcium hydroxide. The malate-forming reaction is then carried out in the usual manner. In this embodiment, calcium may optionally be recycled by use of a lime kiln to convert calcium carbonate by-product of the 2,2'-oxodisuccinate process into calcium hydroxide. Likewise optionally present by way of additional recycle streams may be limited amounts of any one or more of fumarate, malate or 2,2'-oxodisuccinate, within the ranges indicated hereinabove.

## Novel Malic Acid Process

As noted, by simply acidifying the divalent cation-containing product of the malate-making step herein, the divalent cation may be displaced, so that malic acid is obtained. Thus, the invention encompasses a process for the manufacture of malic acid using the above-defined malate-making process, followed by an acidification step. In view of the high purity of the organic component produced in the malate-making process herein, as well as of economic considerations, a malic acid process using the present invention offers considerable advantages. For example, a typical malate-containing product with very low levels of fumarate requires little organic purification.

## Novel Compositions

In its composition aspects, the present invention encompasses the unusual granular, solid products of the malate-making process, the most preferred forms of which are the relatively coarse, easily filterable granule and finer pumpable (in water or mother liquor of the reaction) granule described above. As noted, the processing advantages of these forms of malate are considerable, especially in that they are very readily separated from the aqueous phase of the reaction mixture on one hand, while not forming intractable, large solid masses on the other.

As will be seen from the examples hereinafter, experimental evidence confirms that such granular forms of malate are of unique crystallographic composition, as confirmed by X-ray analysis.

Illustrative of typical organic compositions of the preferred malate product are embodiments 1-7 shown in Table 1. In general, such granular product of the preferred malate-making processes comprises on a dry weight basis, based on malate and maleate and 2,2'-oxodisuccinate and fumarate, from 70% to 99.5% (more preferably from 90% to 99.5%) malate. On the same basis, the organic balance of the composition typically comprises from 0% to 30% (more preferably, less than about 3%) 2,2'-oxodisuccinate and from 0% to 30% (more preferably less than 2%) maleate. The inorganic composition is simply determined on the basis of the amounts of calcium and/or magnesium, and hydroxide excess used. A preferred malate product is further characterized in that it is directly obtainable by said process in alkaline, preferably granular, solid form as noted, further characterized in that it contains a form of calcium malate. Based upon the unique X-ray crystallographic characterization of such product, the invention encompasses a novel composition of matter comprising malate and calcium, said composition comprising a major crystalline component having a characteristic d-spacing of 8.597Å ($2\theta = 10.28°$) as measured using Cu $K_\alpha$ radiation (see the experimental disclosure hereinafter for further details). To be noted is that said alkali, i.e., hydroxide, and calcium cannot be simply removed from the composition without destroying the unique crystalline form thereof; also noteworthy is that d-spacings characteristic of calcium hydroxide are absent or present only at low levels in the crystallographic data. Also encompassed herein is the analogously prepared mixed calcium/magnesium and magnesium forms of such composition.

## Novel 2,2'-oxodisuccinate Process

In addition to the novel malate-making process, the present invention also provides an improved alkaline process for making 2,2'-oxodisuccinate.

Unless otherwise indicated, yields of 2,2'-oxodisuccinate and levels of other organic species herein are expressed as percentages by weight of the total of the organic species present (for example, 2,2'-oxodisuccinate plus maleate plus malate plus fumarate), as analyzed by high-performance liquid chromatography (HPLC).

The above-summarized patent and journal literature provides aqueous processes for manufacturing 2,2'-oxodisuccinate from starting-materials comprising the components I organic starting-material component comprising maleate, II divalent metal cation component and III alkali component. The processes partially convert the organic starting-material component to 2,2'-oxodisuccinate. Though not specifically mentioned in any detail, these processes inherently tend to form by-product, especially fumarate.

The instant invention encompasses an improvement whereby a high 2,2'-oxodisuccinate yield, generally 80% or higher, is secured. The improvement comprises: A. selecting starting-materials which comprise: I an organic component comprising maleate and preformed malate at a maleate:malate mole ratio in the range from 0.7:1 to 2.0:1; II a divalent metal cation component selected from calcium, magnesium and mixtures thereof at a divalent cation component:organic component mole ratio in the range from 0.1:1 to 0.95:1; III an alkali component selected from hydroxide and hydroxide-forming anions; and, additionally, IV a solubilizing monovalent cation component selected from sodium, potassium and mixtures thereof. Also, B. the process

14

is conducted in a fluid, aqueous alkaline mixture of said starting-materials having the following net concentrations by weight:water: no more than 75%; alkali component III, expressed as net excess hydroxide: at least 0.0001%; and solubilizing monovalent cation component IV: at least 1%.

According to the invention, the mixture B is reacted for a period sufficient to attain said 2,2'-oxodisuccinate yield at temperatures in the range from about 20°C to 110°C, and the reaction is then immediately arrested. In general, the following provisions are respected: the total duration of reaction is not less than 12 hours and not more than 400 days; the total time at any temperature above 100°C does not exceed 1.5 hours; the total time at any temperature above 90°C does not exceed 4.5 hours; the total time at any temperature above 80°C does not exceeds 13.5 hours; the total time at any temperature above 70°C does not exceed 1.7 days; the total time at any temperature above 60°C does not exceed 5.1 days; the total time at any temperature above 50°C does not exceed 15 days; the total time at any temperature above 40°C does not exceeds 46 days and the total time at any temperature above 30°C does not exceed 137 days.

It has been found desirable to have only relatively low levels of magnesium or potassium present. Thus, preferred embodiments of the invention include those comprising: A. selecting starting-materials which comprise: I an organic component comprising maleate and preformed malate at a maleate:malate mole ratio in the range from 0.9:1 to 1.8:1; II a divalent metal cation component selected from calcium and mixtures thereof with magnesium, the calcium:magnesium mole ratio ranging from 1.0:0.0 to 0.9:0.1; at a divalent cation component: organic component mole ratio in the range from 0.2:1 to 0.85:1; III an alkali component selected from hydroxide and hydroxide-forming anions; and, additionally, IV a solubilizing monovalent cation component selected from sodium and mixtures thereof with potassium, the sodium:potassium mole ratio ranging from 1.0:0.0 (i.e., no potassium present) to 0.9:0.1 (i.e., a low level of potassium present); and B. conducting the process in a fluid, aqueous alkaline mixture of said starting-materials having the following net concentrations by weight:water: from 25% to 60%; alkali component III, expressed as net excess hydroxide: from 0.0001% to 2%; and solubilizing monovalent cation component IV: from 3% to 20%. This preferred embodiment further involves reacting said mixture at temperatures in the range 20°C to 110°C for a period sufficient to attain the 80%, or higher, 2,2'-oxodisuccinate yield, and arresting the reaction; provided that the total duration of reaction is not less than 12 hours and not more than 240 days and further provided that: the total time at any temperature above 100°C does not exceed 1 hour and the total time at any temperature above 90°C does not exceed 3 hours and the total time at any temperature above 80°C does not exceed 8 hours and the total time at any temperature above 70°C does not exceed 1 day and the total time at any temperature above 60°C does not exceed 3 days and the total time at any temperature above 50°C does not exceed 9 days and the total time at any temperature above 40°C does not exceed 27 days and the total time at any temperature above 30°C does not exceed 81 days.

When using only sodium and calcium as essential monovalent and divalent metal cations, the invention has preferred embodiments which comprise A. selecting starting-materials which comprise I an organic component comprising maleate and preformed malate at a maleate:malate mole ratio in the range from 1.05:1 to 1.7:1, more preferably, from 1.1:1 to 1.6:1; II a divalent metal cation component selected from calcium at a divalent cation component:organic component mole ratio in the range from 0.20:1 to 0.85:1, preferably from 0.25:1 to 0.8:1, very preferably, from 0.35:1 to 0.80:1; III an alkali component selected from hydroxide and hydroxide-forming anions; and, additionally, IV a single solubilizing monovalent cation component, namely sodium; and conducting the process in a fluid, aqueous alkaline mixture of said starting-materials having the following net concentrations by weight:water: from 30% to 50% (more preferably, from 30% to 45%); alkali component III, expressed as net excess hydroxide: from 0.01% to 1.5% (more preferably, from 0.05% to 1%); and solubilizing monovalent cation component IV: from 3% to 16% (more preferably, from 3.5% to 12%). In these embodiments, the fluid, aqueous alkaline mixture of starting-materials is reacted at temperatures in the range 20°C to 110°C for a period sufficient to attain said 2,2'-oxodisuccinate yield, and the reaction is then arrested; provided that the total duration of reaction is not less than 12 hours and not more than 40 days and further provided that: the total time at any temperature above 100°C does not exceed 30 minutes and the total time at any temperature above 90°C does not exceed 1.5 hours and the total time at any temperature above 80°C does not exceed 5 hours and the total time at any temperature above 70°C does not exceed 15 hours (ideally, the total time at any temperature above 65°C does not exceed 1 day) and the total time at any temperature above 60°C does not exceed 1.5 days and the total time at any temperature above 50°C does not exceed 8 days.

As noted in summary, the invention has both isothermal and non-isothermal preferred embodiments. Non-isothermal preferred embodiments include those which consistently afford the higher (e.g., greater than 85%) 2,2'-oxodisuccinate yields.

Embodiments of the invention wherein the components I-IV and water are reacted together non-isothermally include those using the following sequence of steps: (a) an elevated temperature primary reaction procedure of duration 10 minutes to 8 hours, at 50°C to 110°C, contacting the starting-materials to form said fluid, aqueous alkaline mixture and reacting to form a crude product mixture, also fluid, comprising freshly formed 2,2'-oxodisuccinate together with unreacted maleate and malate and immediately; (b) in a lower temperature maturation procedure of duration 1 day to 30 days, reducing the temperature of the crude product mixture of step (a) in one or more steps (e.g., to temperatures in the range from 20°C to 45°C, very preferably in 2 hours or less) while retaining fluidity and continuing to react said crude product mixture, for a period sufficient to chemically combine and form 2,2'-oxodisuccinate from said maleate and malate; thereby increasing the overall proportion of 2,2'-oxodisuccinate present in said crude product mixture while achieving control of the rate of formation of fumarate by-product; and (c) arresting said lower temperature maturation procedure.

In other non-isothermal embodiments, the procedure (b) can be carried out at temperatures above the 20°C-45°C temperature range, but the 2,2'-oxodisuccinate yield maxima will then tend to fall.

More generally, non-isothermal embodiments of the invention need not comprise a sequence of sharply differing temperature steps; for example, continuous cooling from elevated to lower temperatures is perfectly acceptable. Such continuous cooling processes evidently have no extended period of maintaining the reaction at any one temperature, even at the specified high and low temperature limits.

In the non-isothermal preferred embodiments, highly preferred starting-compositions involve A. selecting starting-materials which comprise: I. an organic component comprising maleate and preformed malate at a maleate:malate mole ratio in the range from 1.15:1 to 1.40:1; II. a divalent metal cation component selected from calcium, at a divalent cation component:organic component mole ratio in the range from 0.41:1 to 0.76:1; III. an alkali component selected from hydroxide and hydroxide-forming anions; and, additionally, IV. a solubilizing monovalent cation component, which is sodium; and B. conducting the process in a fluid, aqueous alkaline mixture of said starting-materials having the following net concentrations by weight:water: from 35.0% to 41.0%; alkali component III, expressed as net excess hydroxide: from 0.10% to 0.91%; and solubilizing monovalent cation component IV: from 3.9% to 10.6%.

Even more preferably for maximizing the 2,2'-oxodisuccinate yield, the invention has embodiments wherein step (a) is carried out at elevated temperatures in the range of from 50°C to 110°C and has a duration of from 10 minutes to 5 hours, provided that in step (a), said elevated temperatures are not in excess of 100°C for times greater than 15 minutes and are not in excess of 80°C for times greater than 30 minutes; and wherein step (b) comprises reducing the temperature to lower temperatures in the range of from 20°C to 40°C in a time less than 2 hours and maintaining said lower temperatures; step (b) having a duration of from 1 day to 21 days in total; provided that in step (b), said lower temperatures are not in excess of 36°C for times greater than 7 days and are not in excess of 30°C for times greater than 14 days.

As noted, the chemist has numerous alternative choices of starting-materials. It is preferred to have at least one maleate compound selected from the group consisting of maleic anhydride and maleic acid, together with at least one malate compound selected from the group consisting of malic acid and stereoisomers thereof.

There are several possible sources of sodium for use in the process. It is preferred to use starting-materials which include at least one sodium-containing compound selected from the group consisting of disodium maleate, disodium mat ate, sodium carbonate, sodium bicarbonate and sodium hydroxide.

Common calcium-containing starting-materials include one or more compounds selected from the group consisting of calcium maleate, calcium malate, calcium hydroxide, calcium oxide and calcium carbonate. Certain more unusual chemical forms of calcium-containing starting-material may also be used. For example, a specific form of calcium malate-hydroxide, such as is illustrated in Example 9 hereinafter, is suitable for use in the process.

The starting-materials also generally include at least one compound forming hydroxide anions in water. Such compounds preferably include one or more compounds selected from the group consisting of sodium hydroxide, calcium hydroxide and calcium oxide.

Less desirably, the starting-materials can include various conventional potassium or magnesium salts, such as potassium hydroxide, magnesium hydroxide, or the like.

Whatever the chemical forms of the starting-materials, what is essential herein is to combine them to provide the above-specified ratios and proportions of the components I-IV and water.

One simple full set of starting-materials consists essentially of maleic anhydride, D,L-malic acid, sodium hydroxide and calcium hydroxide. In a preferred process using these starting-materials, the step (a) elevated temperature reaction procedure comprises adding maleic anhydride over a period of 10 to 30 minutes, as portions of solid or as liquid at temperatures above the melting-point but preferably not exceeding 100°C, to a continuously stirred preformed mixture of said D,L-malic acid, calcium hydroxide and sodium hydroxide, the preformed mixture having an initial temperature in the range from 50°C to 85°C, rising exothermically to a maximum of from 100°C to 110°C during the course of maleic anhydride addition, thereby forming a crude product mixture.

In this embodiment, upon ending the above-outlined procedure (a), the crude product mixture is substantially homogeneous and has an organic composition, as a percentage by weight based on 2,2'-oxodisuccinate plus maleate plus malate plus fumarate, of: 2,2'-oxodisuccinate: from 20% to 45%; fumarate: from 1% to 2% and maleate plus malate: from 53% to 79%.

Step (b), i.e., the lower temperature maturation procedure immediately follows step (a) and comprises cooling said crude product mixture to a lower temperature of from 36°C to 40°C within a time of 10 minutes to 2 hours, and storing said crude product mixture at said lower temperature; step (b) having a duration of from 2 days to 7 days in total.

At the end of the above-outlined maturation procedure, the crude product of this embodiment of the process typically has an organic composition comprising, as a percentage by weight based on 2,2'-oxodisuccinate plus maleate plus malate plus fumarate, of: 2,2'-oxodisuccinate: at least 82%; fumarate: from 1.5% to 5.7% and maleate plus malate: from 7.3% to 16.5%.

More than one lower temperature maturation step can be encompassed within the procedure (b); thus the invention has embodiments wherein step (b) comprises: cooling said crude product mixture to a first lower temperature in the range from 36°C to 40°C within a time of 10 minutes to 1 hour, and storing said crude product mixture at said first lower temperature; step (i) having a duration of from 2 days to 7 days; followed immediately by cooling said crude product mixture to a second lower temperature in the range from 25°C to 30°C within a time of 10 minutes to 6 hours, and storing said crude product mixture at said second lower temperature; step (ii) having a duration of from 7 days to 14 days.

At the end of such a two-step or multi-step procedure (b), the crude product of this embodiment has the following organic composition, as a percentage by weight based on 2,2'-oxodisuccinate plus maleate plus malate plus fumarate, 2,2'-oxodisuccinate: at least 90% (typically, 90%-95%); fumarate: from 2% to 6% and maleate, mat ate or mixtures thereof: to 100%.

In general, it is essential to arrest all organic product and by-product-forming chemical reaction at the end of the lower temperature maturation procedure, to avoid the 2,2'-oxodisuccinate content of the crude product falling after it reaches the maximum made possible by the process. When the 2,2'-oxodisuccinate content falls, the levels of fumarate by-product concurrently begin to increase. Thus, in a preferred embodiment of the invention, immediately after the lower temperature maturation procedure, reaction (b) is arrested by (c) treating said crude product mixture with a warm aqueous mixture of sodium carbonate and sodium bicarbonate, thereby precipitating calcium carbonate.

Provided that the practitioner respects the overall temperature-time limitations given supra, and thereby avoids over-extended periods of reacting the starting-materials at particular temperatures, he may flexibly opt to carry out one or more lower temperature maturation steps in a procedure of type (b), based upon monitoring the organic composition of the reaction mixture or crude product mixture.

When carrying out such monitoring, for example by high-performance liquid chromatography (HPLC), monitoring of 2,2'-oxodisuccinate level or of fumarate level is quite sufficient.

Naturally, the practitioner will recognize that fumarate is an impurity common in maleate and malate feedstocks (as well as being a by-product, as noted, in the instant process). Therefore, when monitoring fumarate levels in the instant process, the practitioner will begin the process using starting-materials of known fumarate content.

The invention thus has preferred embodiments which include a process wherein the starting-materials have a known fumarate level, e.g., when they comprise no more than 0.01 moles of fumarate impurity per mole of said maleate plus said preformed malate, and wherein step (a) is carried out at elevated temperatures in the range from 50°C to 110°C, provided that in step (a), said elevated temperatures are not in excess of 100°C for times greater than 15 minutes and are not in excess of 80°C for times greater than 1 hour; and wherein step (b) comprises reducing the temperature to lower temperatures in the range of from 20°C to 40°C, provided that in step (b), said lower temperatures are not in excess of 36°C for times greater than 7 days and are not in excess of 30°C for times greater than 14 days; step (a) being ended and step (b) being undertaken at any time corresponding with a net increase in fumarate level, based upon HPLC-analysis of the crude product mixture, in the range from 0.5% to 5%.

Even more rigorously limiting increases in fumarate level by analytical monitoring of the process further increases yield. To illustrate, the above embodiment can be modified by ending step (a) and undertaking step (b) at any time corresponding with a net increase in fumarate level, based upon HPLC-analysis of the crude product mixture, in the range from 0.5% to 2.5%; and wherein step (b) is ended and step (c) is undertaken immediately upon reaching an HPLC-based 2,2'-oxodisuccinate yield of at least 85%.

The practitioner of the invention may not wish to realize the maximum 2,2'-oxodisuccinate yields, preferring to save time and secure good 2,2'-oxodisuccinate yields in a typical range 80%-85%. In this event he may opt to carry out step (a) at elevated temperatures in the range from 70°C to 110°C, in a period of from 4 hours to 5 hours, provided that in step (a), said elevated temperatures are not in excess of 100°C for times greater than 15 minutes and are not in excess of 80°C for times greater than 30 minutes. In step (b), the temperature is reduced to lower temperatures in the range of from 50°C to 59°C in a period of from 15 minutes to 1 hour, and the lower temperatures are maintained, step (b) having a duration of from 7 hours to 20 hours in total.

In contrast, another preferred embodiment of the invention having better yields but taking more time, is as follows: step (a) is carried out at elevated temperatures in the range from 75°C to 110°C, in a period of from 20 minutes to 1 hour, provided that in step (a), said elevated temperatures are not in excess of 100°C for times greater than 15 minutes and are not in excess of 80°C for times greater than 30 minutes; and step (b) comprises reducing the temperature to lower temperatures in the range of from 35°C to 45°C in a period of from 15 minutes to 1 hour, and maintaining said lower temperatures; step (b) having a duration of from 48 hours to 240 hours in total; whereby a 2,2'-oxodisuccinate yield of at least 85% is secured.

A preferred procedure to be followed when using calcium carbonate as a calcium source, i.e., as a calcium starting-material, in the instant process is as follows: preformed malic acid, sodium hydroxide, a maleate reactant selected from maleic anhydride, maleic acid and mixtures thereof, and a calcium reactant selected from calcium carbonate and mixtures thereof with calcium hydroxide are reacted according to the immediately consecutive steps: (i) mixing calcium carbonate, water, malic acid and a proportion of said maleate reactant, allowing complete evolution of carbon dioxide and forming an acidic mixture; (ii) adding sodium hydroxide or a mixture thereof with calcium hydroxide, to the acidic mixture of step (i), forming a sodium cation-containing alkaline mixture; (iii) in a period of duration 1 hour or less, adding the remainder of said maleate reactant to the stirred sodium cation-containing alkaline mixture of step (ii), at temperatures in the range from 75°C to 110°C, having at the end of the step (iii) addition a net hydroxide excess of component III $M_{OH}$; (iv) in a period of duration 1 hour or less, cooling the mixture formed in step (iii) to a temperature in the range from 35°C to 45°C; (v) at said temperature in the range from 35°C to 45°C, continuing to react the mixture of step (iv); the duration of step (v) being from 48 hours to 240 hours, whereby a crude product having a HPLC yield of at least 80% 2,2'-oxodisuccinate is secured; and (vi) diluting the product of step (v) with water and precipitating calcium carbonate therefrom; thereby arresting the step (iv) reaction and depleting the level of calcium; provided that in steps (iii) and (iv) together; the total time at any temperature above 100°C does not exceed 15 minutes; the total time at any temperature above 90°C does not exceed 30 minutes; the total time at any temperature above 80°C does not exceed 2 hours; the total time at any temperature above 70°C does not exceed 6 hours; and the total time at any temperature above about 65°C does not exceed 12 hours; and provided that for each mole of preformed malic acid reacted, the total molar amount of maleate reactant is 1.1 to 1.6 moles; the total molar amount of calcium reactant, is from 0.9 to 1.65 moles; the total molar amount of sodium hydroxide, is from 0.92 to 3.7 moles; the net hydroxide excess in step (iii), $M_{OH}$, is from 0.02 to 0.3 moles; and further provided that for each mole of preformed malic acid reacted (i.e., charged in the reactor), the total net amount of water added in steps (i), (ii) and (iii) together, allowing for evaporation losses, is no less than 189 grams and no more than 282 grams.

The above procedure has advantages in that calcium carbonate can be recycled by (vii) filtering the mixture of step (vi) to secure a filter-cake and (viii) using the filter-cake of step (vii) as recycled source of calcium carbonate in step (i).

Returning to the isothermal reaction procedures that can also be practiced in accordance with the invention, the term "isothermal" is deserving of comment. Isothermal processes herein are those in which substantially constant temperature is maintained, from the time at which at least some of each of the essential components I-IV is present in a fluid, aqueous, alkaline mixture (i.e., from the time at which it is possible for 2,2'-oxodisuccinate formation to occur), until the time at which reaction is arrested as defined hereinabove. The term "substantially constant" in this context makes allowance for practical considerations, such as precision of conventional process control equipment, e.g., thermostats.

Isothermal processes herein include those in which the divalent cation component:organic component mole ratio is in the range from 0.40:1 to 0.75:1, and in which the process is conducted at a concentration of water in the range from 35% to 41% and comprises reacting said mixture at a temperature in the range from 50°C to 68°C for a period of from 48 hours to 240 hours, then arresting the reaction.

18

Other isothermal processes herein, such as those carried out at 40°C to 49°C, are also effective, but are of longer duration.

Crude product mixtures in the present process are unique. After carrying out the instant process using the details of temperature and time or HPLC monitoring given herein, the practitioner may turn to very simple characteristics of the crude product mixture, such as visually, optically or, especially, rheologically measurable characteristics of the crude product mixture, as they change during the process, for aid in monitoring the process.

Crude product mixtures made by reacting the starting-materials according to the invention have the following characteristics as soon as they are sufficiently reacted to commence a lower-temperature maturation procedure such as (b) described above: taking the starting-materials, maleic anhydride and an aqueous alkaline mixture of D,L-malic acid, sodium hydroxide and calcium hydroxide as a simple illustration, the mixture formed upon adding some maleic anhydride to the aqueous alkaline malate-containing mixture in step (a) is a two-phase mixture consisting of a liquid phase, in major proportion, and a solid phase, in minor proportion. The proportion of the solid phase decreases and the viscosity of the liquid phase increases during addition of the remaining maleic anhydride and thereafter, so that, at the end of step (a), a crude product mixture is formed which is pumpable, i.e., fluid, and which appears substantially homogeneous to the eye.

Adding viscosity data, the Brookfield relative viscosity of the crude product mixtures in the process typically increases during the lower temperature maturation procedure (b) from an initial value of less than 100 centipoise to a final value at the end of procedure (b) which is in the range 1000 centipoise to 100,000 centipoise, as measured under Brookfield test conditions of 22°C/20 r.p.m. Preferably, while the crude product mixture generally remains pumpable, the viscosity increase during the process is of 3000 centipoise or more. Also, the crude product mixtures typically fail to separate into visually distinct layers upon standing unstirred, e.g., at test temperatures of 60°C for a test period of 48 hours.

Expressing the hereinbefore described ratios and proportions of reactants in certain preferred embodiments of the instant process on a weight basis is helpful in allowing the practitioner of the invention to carry out the process with excellent results. Thus, the invention encompasses conducting the process in a fluid, aqueous alkaline mixture of said starting-materials, the amounts of which are calculated to deliver by weight, expressed on a fully hydrolyzed and neutralized basis assuming no 2,2'-oxodisuccinate or fumarate formation and no maleate → malate interconversion: water: from 30.0% to 45.0%; net excess hydroxide: from 0.05% to 1.0%; sodium: from 3.4% to 13%; calcium: from 4.5% to 13%; maleate: from 19.1% to 28.4% and preformed malate: from 16.5% to 26.8%.

When the process is carried out with inclusion of recycled fumarate and/or 2,2'-oxodisuccinate (for example, entrained in a recycled calcium carbonate filter-cake) or with inclusion of fumarate and/or 2,2'-oxodisuccinate starting-material impurities (for example, as can be present as fumaric acid in D,L-malic acid, or as can be present as calcium fumarate in calcium malate), then the above weight percentages should be adjusted to allow for their presence. Typically, recycled 2,2'-oxodisuccinate levels at the start of the process do not exceed 2%. Likewise, fumarate recycle or fumarate impurity levels at the start of the process typically do not exceed 2%. Naturally, it is preferred to use fumarate-free starting-materials.

Conventional maleate, malate and fumarate chemistry is well reviewed in "Maleic Anhydride", B. C. Trivedi and B. M. Culbertson, Plenum Press, N.Y., 1982 and in Kirk Othmer, Encyclopedia of Chemical Technology, 3rd Ed., 1981, Vol. 13, pp. 103-121 and Vol. 14, pp. 770-793, all incorporated herein by reference.

Unless otherwise indicated, all ratios herein are mole ratios and all percentages are by weight.

The invention is further illustrated by the following non-limiting examples:

Examples Illustrating Novel Solid-Form Malate as Calcium or Magnesium Salts

EXAMPLE I

Maleic anhydride (29.4 g, 0.3 moles, Aldrich) is dissolved in distilled water by heating to 85-90°C in an open preweighed Hastalloy C autoclave (Parr Instrument Co., Model 4561, 300 cc) and stirring. The solution is cooled by placing the autoclave in an ice-bath. When a temperature of 60-80°C is reached, calcium hydroxide (33.3 g, 0.45 moles, Aldrich, A.C.S.) addition is started. A thick paste forms, which thins significantly as addition of calcium hydroxide progresses. When all the calcium hydroxide is added, the total reaction weight is corrected by adding water to a reaction mixture weight of 192.0 g. The water content is calculated as 70.2%. The autoclave is sealed, placed in a 165°C thermostat-equipped oil bath and stirred. After bringing the reaction mixture to equilibrium with the oil bath temperature, further stirring is continued for 4.5 hours (the reaction time as defined herein). Note that the oil bath and autoclave are previously calibrated; it is shown that an oil bath temperature of 165°C corresponds with an internal autoclave temperature of 145°C. Pressure reaches a maximum of 45 psig (0.31 MPa; where 1 psi = 0.006895 MPa). The autoclave is cooled in water, opened, and weighed. The weight is within 2 g; insignificant leakage of the autoclave is thereby demonstrated. The contents of the autoclave are comprised primarily of a granular product, white in color and odorless. No off-odors are detected. (When very high temperatures, 185°C or more, are used, the product acquires trace odor and trace impurity peaks in HPLC analysis.) The autoclave also contains some finer solids and a thin crust on the internal walls. All contents are readily scraped out and rinsed into a WARING BLENDOR. Using distilled water, the sample weight is made up to 320 g and blended for the purpose of homogeneous sampling for analysis. (For workup, simply acidify with sulfuric acid and separate calcium sulfate leaving a solution of malic acid or, simply filter and air dry; or, use for making 2,2'-oxodisuccinate, as desired.) 0.4 g samples of the Waring-blended product mixture are quenched in 100 cc lots of 0.1N $H_2SO_4$ with vigorous shaking, and the samples are submitted for HPLC analysis. Analysis indicates that by weight % of organic components, the product comprises 94.7% malate. 2.1% 2,2'-oxodisuccinate oxodisuccinate and 3.2% fumarate are copresent. No maleate is detected.

EXAMPLES II - XV

Using the procedure of Example 1, together with preferred calcium/maleate mole ratios in the range 1.4:1 - 1.5:1 moles/mole, temperature, pressure and time vs organic composition of the reaction mixture are studied. Results are tabulated below in Table 2.

TABLE 2

| Example No. | Calcium/ Maleate Mole Ratio | Maximum Pressure (psig) Observed | Oil Bath to °C | Reaction Mixture to °C | Time Hours |
|---|---|---|---|---|---|
| II | 1.5:1 | not measured | 135 | 120 | 16 |
| III | 1.5:1 | 35 (0.24 MPa) | 145 | 130 | 8 |
| IV | 1.5:1 | not measured | 155 | 140 | 4 |
| V | 1.5:1 | not measured | 155 | 140 | 10 |
| VI | 1.5:1 | not measured | 155 | 140 | 1 |
| VII | 1.5:1 | not measured | 155 | 140 | 2.4 |
| VIII | 1.5:1 | 40 (0.28 MPa) | 155 | 140 | 4 |
| IX | 1.5:1 | 42 (0.29 MPa) | 155 | 140 | 4 |
| X | 1.5:1 | 35 (0.24 MPa) | 155 | 140 | 4 |
| I | 1.5:1 | 45 (0.31 MPa) | 165 | 145 | 4.5 |
| XI | 1.5:1 | 42 (0.29 MPa) | 165 | 145 | 4.25 |
| XII | 1.4:1 | 56 (0.39 MPa) | 165 | 145 | 6 |
| XIII | 1.4:1 | 80 (0.55 MPa) | 165 | 145 | 6 |
| XIV | 1.5:1 | 115 (0.79 MPa) | 185 | 165 | 1 |
| XV | 1.5:1 | 120 (0.83 MPa) | 200 | 180 | 2 |

TABLE 2 (continued)

| Example No. | Weight % (HPLC) Malate | Weight % (HPLC) ODS* | Weight % (HPLC) Fumarate | Weight % (HPLC) Maleate |
|---|---|---|---|---|
| II | 88.8 | 8.2 | 3.0 | 0 |
| III | 92.9 | 2.5 | 4.6 | 0 |
| IV | 94.4 | 2.1 | 3.4 | 0.1 |
| V | 95.7 | 1.4 | 2.9 | 0 |
| VI | 30.6 | 12.9 | 7.8 | 48.7 |
| VII | 70.6 | 14.3 | 10.6 | 4.5 |
| VIII | 94.1 | 3.5 | 2.4 | 0 |
| IX | 94.2 | 2.9 | 2.9 | 0 |
| X | 93.3 | 3.2 | 3.5 | 0 |
| I | 94.7 | 2.1 | 3.2 | 0 |
| XI | 93.5 | 2.7 | 3.9 | 0 |
| XII | 97.8 | 0.4 | 1.8 | 0 |
| XIII | 98.1 | 0.7 | 1.1 | 0 |
| XIV | 94.9 | 2.5 | 2.6 | 0 |
| XV | 96.0 | 2.4 | 1.6 | 0 |

(All of Examples I-XV use 70-72% weight % water)

*ODS = 2,2'-oxodisuccinate

Example II confirms that even at relatively low temperatures in the preferred range, results quite different from those of Berg are obtained: only 8.2% 2,2'-oxodisuccinate is present. Examples VI, VII and VIII are particularly instructive in that they demonstrate that mixtures having appreciable proportions of 2,2'-oxodisuccinate, maleate and fumarate can be converted to malate in high yield. The remaining Examples through XV generally illustrate high yields with low residual levels of fumarate, and the varying times, temperatures and pressures herein.

EXAMPLE XVI

Air-dried samples of the solid product of Example XV are gently ground, using an agate pestle and mortar, and packed in a sample planchette. X-ray diffraction data are collected using a computer-controlled STOE diffractometer with diffracted-beam graphite monochromator; $CuK_\alpha$ radiation; X-ray generator at 40 kV/37mA; scan range 1-70° in $2\theta$ at a rate of 0.04/step and counting time 4 seconds/step.

The X-ray diffraction pattern has a major spacing of 8.597 Å ($2\theta$ = 10.28°) inconsistent with any known crystal form of calcium malate, calcium malate hydrate, malic or maleic acids, calcium hydroxide or calcium oxides in the JCPDS International Centre for Diffraction Data, 1601 Park Lane, Swarthmore, PA, 19081 (formerly Joint Committee on Powder Diffraction Standards) reference files.

EXAMPLE XVII

Using the procedure of Example I at water content ranging from 70% to 75% by weight of the reaction mixture, a study of calcium/maleate ratios is undertaken. The optimum combinations of high improved yield of malate and easily handled product are obtained at calcium/maleate ratios greater than 1.3:1 but less than 1.7:1, especially at ratios of 1.5:1.

EXAMPLE XVIII

Using the procedure of Example I and varying the water content of the reaction mixtures from 40% to 70% by weight, improved results, especially in that reduced fumarate by-product levels are obtained, occur when the water content is from 50% to 65 wt. %. As illustrated at calcium/maleate mole ratio of 1.2:1, fumarate by-product levels rise significantly at water contents below 50% and above 65%.

EXAMPLE XIX

A procedure similar to that of Example I is employed, except that calcium carbonate is used to partially neutralize a solution of maleic acid; $CO_2$ evolution is complete prior to addition of calcium hydroxide in an amount selected to provide the same overall calcium/maleate and excess hydroxide: maleate ratios as used in Example I. Yields of malate similar to those of Example I are obtained.

The modified procedure demonstrates that calcium may be recycled as the carbonate and used in a pre-neutralizing step in the instant invention.

EXAMPLE XX

A study is undertaken of the impact of varying ratios of magnesium, (up to and including total replacement of the calcium hydroxide by magnesium, used as the hydroxide), to calcium in a procedure otherwise similar to that of Example I. Surprisingly, low levels of magnesium hydroxide give unexpected improvements in yield and give a unique granular product somewhat finer than that of Example XV. In one example, embodiment 3, Table 1, using 1.45 moles $Ca(OH)_2$ and 0.05 moles $Mg(OH)_2$ per mole maleate, a reaction temperature of 160°C, a reaction time of 6 hours, a maximum pressure of 82 psig (0.57 MPa) and a water content of 70%, malate yield of 99.2% is obtained. Corresponding levels of 2,2'-oxodisuccinate, fumarate and maleate are 0.2%, 0.6% and 0% respectively.

EXAMPLE XXI

(Not in accordance with the invention.)

Dry, granular "calcium malate" prepared according to the procedure of any one of Examples I-XV (excluding VI and VII) is finely ground using a pestle and mortar and converted to 2,2'-oxodisuccinate as follows. Taking the product of Example I for the purposes of illustration, 66.24 g (equivalent to 0.3 moles content of calcium malate and 0.15 moles content of $Ca(OH)_2$ of the ground material, 34 cm$^3$ $H_2O$. and 38.8 g, (0.48 moles) NaOH as 50% aqueous solution, (Fisher) are mixed together in a glass or stainless steel reaction vessel equipped with a stirrer, reflux condenser and heating oil bath. Powdered calcium hydroxide (2.44 g, 0.033 moles, Aldrich) is mixed in. The reaction vessel Is heated, with stirring, to a temperature of 70-80°C; at which point a milky or chalky suspension is observed. Crushed, powdered maleic anhydride (36.5 g, 0.37 moles, Aldrich) is added over 5-10 minutes in small portions. Thought the reaction is exothermic and may briefly reach temperatures close to reflux temperature, good stirring and thermostatting of the heated bath ensures that the mixture equilibrates rapidly to the 70-80°C. range. During the last 3 minutes of maleic anhydride addition, a light amber homogeneous mixture, substantially clear to the eye, is formed. For best yields (assuming that the maturation process illustrated in Example XXII hereinafter is not used), the reaction is continued for a reaction time of from 5 to 6 hours, this time being measured from completion of the maleic anhydride addition. The organic composition of the crude product corresponds to a 2,2'-oxodisuccinate content based on HPLC peak areas (Distribution Analysis) of 74.5%; 9.7% malate, 9.5% maleate and 6.3% fumarate are copresent. Higher 2,2'-oxodisuccinate levels and lower fumarate levels are obtained in an analogous preparation based on product of Example XX having low levels of magnesium present.

EXAMPLE XXII

(Modification of XXI in order to comply with the invention.)

The procedure of Example XXI is reproduced, with the single exception that as soon after completion of the maleic anhydride addition as the homogeneous reaction mixture is formed, the mixture is cooled to 30-50°C. The mixture is maintained at the new, lower temperature for a period of from 12 hours to 21 days.

HPLC analysis now gives the following results: 88.0% 2,2'-oxodisuccinate; 3.0% maleate; 3.0% malate and 6.0% fumarate.

EXAMPLE XXIII

A procedure similar to that of Example I is used, with the following reactants:
0.9 moles maleic anhydride,
0.1 moles fumaric acid,
1.3 moles Ca(OH)$_2$,

The water content is 70%, reaction temperature (oil bath) is 185°C and reaction time is 6 hours. A fine granular suspension containing by way of organic components (as analyzed by HPLC) 99.0% malate, 0.5%, 2,2'-oxodisuccinate, 0.5% fumarate and 0% maleate is obtained. The inorganic composition is as determined on the basis of the reactants used.

EXAMPLE XXIV

Conversion of product of Example XXII to tetrasodium 2,2'-oxodisuccinate

The reaction product mixture of Example XXII is treated with excess aqueous NaHCO$_3$ Na$_2$CO$_3$ giving a mixture having a pH of 10. This is heated for 4 hours at 70°C with stirring, producing tetrasodium 2,2'-oxodisuccinate and inorganic precipitate which is separated by filtration. The solution of product is conventionally dried.

EXAMPLE XXV

Conversion of product of Example I to malic acid

The product of Example I is acidified with excess aqueous H$_2$SO$_4$, liberating malic acid.

EXAMPLE XXVI

Malate-making process (a) in presence of a limited proportion of tetrasodium 2,2'-oxodisuccinate (such as may be recycled from product of process steps (b) or (c)

The procedure of Example I is reproduced using the following reactants:
28.8g (0.294 moles) maleic anhydride,
3.62g of a 28% aqueous solution of tetrasodium 2,2'-oxodisuccinate, (yielding a total of 0.3 moles of component 1), 123.9g of added water, and 33.3g (0.45 moles) calcium hydroxide; with a water content of 70% in total, a reaction temperature of 185°C, and a reaction time of 6 hours. A fine granular suspension is obtained. This has excellent ease of handling, and contains by way of organic components as analyzed by HPLC: 98.4% malate, 0.4% 2,2'-oxodisuccinate, 1.2% fumarate and 0% maleate. The inorganic composition is determined on the basis of the reactants used.

EXAMPLE XXVII

2,2'-oxodisuccinate-making process in detail

Equipment:

The following equipment is made ready: open-topped reactor drum of 316 stainless steel (SS), 60 liter capacity, equipped with removable plastic cover and with twin rotor turbine stirrer driven by a shaft connected above to a compressed air motor; doublewall, insulated vessel of SS or equivalent Dewar vessel having 200 liter capacity, suitable for substantially complete immersion of the reactor drum, with a good space, at least 8 inches, around the reactor drum as well as between the bottom of the reactor drum and the bottom inner wall of the Dewar vessel. The Dewar vessel has steam and hot and cold water inlets and outlets. Also provided are steam sprayer coils; 3 kW electrically powered and thermostatically controlled immersion heater; immersible water circulation pump; and removable cover for minimizing evaporation of heating water, capable of covering the annular space between the reactor drum and the inner wall of the

23

Dewar vessel; thermometers both for reactor drum and Dewar vessel.

Assembly:

The steam sprayer coils are placed around the reactor drum and the drum and surrounding coils are placed in the Dewar vessel. The remaining equipment is assembled so as to allow rapid but controlled heating of the reactor drum. Such heating involves any of: passage of hot water through the Dewar vessel, electrical heating and recirculation of water in the Dewar vessel, or direct steam heating of the reactor drum by steam sprayed through the sprayer coils onto the outer walls and bottom of the reactor drum. The equipment is also assembled so as to allow rapid but controlled cooling, by passage of tempered or cold water into the Dewar vessel with drainage of hot water, or by addition of ice to the water in the Dewar vessel.

Precontacting of alkali, malate, sodium and calcium:

A 50% aqueous solution of sodium hydroxide (total weight 17520 g, contains 219 moles NaOH, Fisher) is added with stirring to distilled water (14360 g). The caustic solution is then heated to 50°C. Over a period of 9 minutes, granular D,L-malic acid (13400 g, 100 moles, Aldrich, 99%) is added in portions to the vortex of the stirred caustic solution, cooling as necessary to maintain a temperature in the range 80°C - 95°C. Over a period of 6 minutes, powdered calcium hydroxide (9176 g, 124 moles, Aldrich, 98% +, ACS grade) is now added in portions to the vortex of the alkaline Na/Ca malate mixture, maintaining the temperature. Over a period of 20 minutes, stirring is continued without substantially altering the temperature. At the end of this period, the temperature of the alkaline Na/Ca malate mixture is 85°C.

(a) Contacting and primary reaction:

Over a 14 minute period, maleic anhydride (12544 g, 128 moles, Aldrich, 99%, briquettes, freshly crushed) is added to the vortex of the stirred alkaline Na/Ca malate mixture at a steady rate. During this period, an exotherm carries the temperature of the reaction from 85°C to a maximum in the range 100°C-110°C, more typically 103°C - 105°C. Also during this period, within 10 minutes of starting to add maleic anhydride, the visual appearance of the reaction mixture changes from that of a chalky suspension typical of calcium salts to that of a homogenous but somewhat opaque and honeylike crude product mixture.

At the end of the maleic anhydride addition period, cooling is applied over a period of 16 minutes bringing the reaction temperature to 76°C - 78°C. One operator now characterizes the crude product mixture, while a second operator continues the process.

The first operator finds that the viscosity has increased relative to the starting-point of maleic anhydride addition. A first high performance liquid chromatography (HPLC) sample, is taken by the first operator at the 30 minute point. See the analytical protocol hereinafter for details of the HPLC analysis. The time = 30 minute sample of the crude product mixture contains about 43.6% by weight in total of maleate plus malate plus 2,2'-oxodisuccinate plus fumarate, calculated as the anions. The relative weight percentages of the anions are: maleate, 28.3%; malate, 27.4%; 2,2'-oxodisuccinate, 42.5%; fumarate, 1.8%; i.e., as defined herein, the 2,2'-oxodisuccinate yield at this stage is 42.5%. The total weight of the crude product mixture is 63800 g. A pH meter reading of 10.7/78°C is obtained using a combination electrode (Corning X-EL Cat. No. 476262, calibrated against a pH 10 buffer at 25°C and temperature compensated for readings at 78°C). Note that the meter readings under these conditions of concentration, especially sodium ion concentration, as well as of temperature, are useful numbers for process monitoring, provided that the electrodes used are new or otherwise proven reliable; however, the meter readings are not true pH values.

The second operator ensures that as of the end of the above-mentioned 16 minute cooling period, the crude product mixture is continuously stirred at 78°C for 4.5 hours. At this stage, HPLC analysis shows that the 2,2'-oxodisuccinate level has reached 72% with 4% fumarate and approximately balanced levels of unreacted maleate and malate also present. The following maturation procedure is now immediately carried out.

(b) Maturation:

Rapidly (less than 2 hours, more preferably, less than 1 hour), the temperature of the crude product mixture is reduced to approximately 40°C. The crude product mixture is held at this 40°C temperature for a total maturation time of 139 hours, measured from the start of cooling in the maturation procedure. During

24

maturation, the crude product mixture is infrequently stirred. (Typically, one-hour periods of stirring precede HPLC sampling on a once-daily basis; the stirring ensures representative sampling.) The 2,2'-oxodisuccinate yield at the end of the 139 hour maturation is 82%, with less than 6% fumarate by-product.

A second maturation stage is undertaken. The crude product mixture is cooled to 27°C within 6 hours or less (on small scale versions of this experiment, cooling times can be as short as 10 minutes) and maintained at that temperature, with once-daily stirring prior to HPLC sampling. The second maturation stage has a total duration of 72 hours. The 2,2'-oxodisuccinate yield is now 85.3%, with 6.8% fumarate by-product. The crude product mixture as sampled at this stage has a density of 1.50 g/cm$^3$ at 35°C.

A third maturation stage is now undertaken. The crude product mixture is further cooled to 23°C and maintained at that temperature, with once-daily sampling as before. The third maturation stage has a total duration of 95 hours. The 2,2'-oxodisuccinate yield is now 87%; 7% fumarate by-product is present.

Viscosity characterization of crude product mixture:

Brookfield viscosity readings are obtained at t = 48 hours / 40°C / 50 r.p.m.: 1600 cps and t = 288 hours / 22°C / 50 r.p.m.: 7300 cps.

(c) Arresting the Reaction; Inorganic Workup:

To avoid decreasing the 2,2'-oxodisuccinate yield and increasing the fumarate by-product levels, the reaction is arrested, and simultaneously, calcium levels are depleted, replacing calcium with sodium cations and producing a 2,2'-oxodisuccinate product directly useful as a laundry detergent builder, as follows: The crude product mixture is diluted with 10000 g distilled water at 25°C, is then siphoned out of the reactor drum, and is further diluted with 76000 g distilled water. Sodium carbonate (14480 g, 136.6 moles, Fisher) and sodium bicarbonate (1036 g, 12.3 moles, Fisher ACS grade) are added at 70°C (temperatures such as this help calcium carbonate precipitation in filterable form). A pH meter reading of 9.94 / 72°C is obtained. The mixture is stirred for 5 hours at 70°C. Upon cooling to ambient temperature, the mixture is filtered through two layers of filter paper (E-D Corp., No. 613-20, 60 cm diameter). (The filter cake may be recovered and re-used; see Example 3). The filtrate is evaporated to a volume of 80 liters. A polishing filtration through coarse frit glass Buchner filters is carried out. The stable, liquid-form finished product 2,2'-oxodisuccinate builder weighs 92117 g (92.1 kg) and analyzes, by weight of organics, 87.1% 2,2'-oxodisuccinate (i.e., yield of the process is 87.1%); 6.7% fumarate, 3.2% maleate and 3.1% malate are also present. The organic composition of the product is not significantly affected by the workup.

EXAMPLE XXVIII

Equipment: Small-scale version of the equipment used in Example XXVII.

Starting-Materials:

| | |
|---|---|
| Distilled Water: | 108.8 g |
| 50% Aqueous NaOH: | 161.6 g (2.02 moles NaOH) |
| D,L-Malic Acid: | 134.1 g (1.0 mole) |
| Ca(OH)$_2$ | 94.7 g (1.24 moles) |
| Maleic Anhydride: | 117.6 g (1.20 moles) |
| Total Weight: | 616.8 g |
| Weight Percentage of maleate ($C_4H_2O_4$) plus malate ($C_4H_4O_5$): | 43.6% |

(a) Contacting and Primary Reaction:

Precontacting:

The 50% aqueous sodium hydroxide is added to the distilled water, with stirring, at 60°C- 70°C.

The D,L-malic acid, as granules, is stirred in while the temperature is maintained in the range 60°C-70°C.

The calcium hydroxide is now added, maintaining the temperature and stirring. This precontacting procedure achieves formation of a preformed mixture containing the essential sodium, calcium, malate, alkali (hydroxide) and water components. This mixture is weighed. Now, only the maleate component need be added.

Final Contacting:

Over 20 to 25 minutes, the temperature initially 60°C, freshly crushed maleic anhydride, in the above-indicated total amount, is added to the preformed mixture of the precontacting procedure. The maleic anhydride is added at a steady rate, with stirring. The reaction mixture is briefly allowed to heat exothermically to a temperature of 80°C during the maleic anhydride addition. The weight is checked and a small amount of water is added, to compensate for evaporation loss during the maleic anhydride addition.

(b) Maturation:

During the procedure which follows, the reaction is conducted at approximately constant weight by adding small amounts of water to compensate for evaporation losses as the reaction progresses.

Stirring is maintained and the crude product mixture of step (a) is cooled over a one hour period to a temperature of 40°C. The reaction vessel and crude product mixture are transferred to an oven, thermostatically controlled at 40°C. The unstirred contents of the reaction vessel are maintained at 40°C for a 167-hour period. In an additional maturation stop, the reaction vessel and crude product mixture are removed from the oven, are cooled to 25°C over 1 hour and are then stored without stirring for an additional 168 hours.

(c) Arresting the Reaction; Inorganic Workup:

The procedure (c) of Example XXVII is used. The amounts of water, sodium carbonate and sodium bicarbonate are reduced in proportion with the scale and stoichiometry of the process.

HPLC analysis of the product of step (b) reveals that it has a 2,2'-oxodisuccinate yield of 92% with fumarate by-product levels of 4%. 4% of unreacted maleate and malate are present. The organic analysis is unchanged after workup.

EXAMPLE XXIX

The equipment described in Example XXVII is used.

Precontacting of malate, a little maleate and calcium by preneutralization with calcium carbonate:

To a solution at 70°C comprising water (18000 g), maleic acid (1622 g, 14 moles) and D,L-malic acid (6700 g, 50 moles) in an open reaction vessel is added slowly over 30 minutes, with stirring, a filter cake, such as produced in Example XXVII, containing calcium carbonate (6200 g, 62 moles). Carbon dioxide and a significant fraction of entrained water are allowed to boil off for 30 minutes after completing the addition.

Addition of Sodium Hydroxide:

50% aqueous sodium hydroxide (8760 g, 109.5 moles NaOH) is slowly added to the mixture with stirring. During the sodium hydroxide addition, further loss of water is permitted. The sodium cation-containing alkaline mixture now contains 12917 g water.

Contacting of the bulk of the maleate and primary 2,2'-oxodisuccinate-forming reaction:

Over a 20 minute period, molten maleic anhydride at 65°C (4900 g, 50 moles) is added to the vortex of the stirred mixture at a steady rate. During this period, an exotherm carries the temperature of the reaction from 85°C to a maximum in the range 103°C-105°C and cooling is applied so that at the end of the 20 minute period, reaction temperature is 76°C-78°C.

Maturation Procedure:

Rapidly (in less than 1 hour), the temperature of the crude product mixture is reduced to 40°C. The crude product mixture is held at 40°C for a total duration of the maturation procedure of 240 hours. The crude product mixture now has a 2,2'-oxodisuccinate yield of 83%. The product workup is as in Example XXVII.

26

EXAMPLES XXX, XXXI, XXXII and XXXIII

Common Procedure: (throughout, evaporation of water is prevented or the water level is adjusted to keep an approximately constant water content of the reaction. Also, HPLC analysis samples are taken at regular intervals. See Example XXVII and HPLC Analysis Protocol hereinafter for further details).

Equipment: small-scale version of the equipment used in Example XXVII.

(a) Contacting and Primary Reaction:

Precontacting:

Add 50% aqueous sodium hydroxide to distilled water with stirring at 60°C-70°C.
Stir in D,L-malic acid while holding the mixture at a temperature in the range 80°C-90°C.
Add calcium hydroxide with stirring at about the same temperature.
Stir for 10 minutes.

Final Contacting:

Over 10 to 15 minutes at 85°C, add maleic anhydride at a steady rate with stirring. Allow exothermic heating of the reaction mixture from 85°C to 105°C at least for a brief period during the maleic anhydrlde addition.

Stir an additional 15 minutes, during which reaction temperature is rapidly brought down to $T_r$.

The above-mentioned 10-15 minutes of maleic anhydride addition plus 15 minutes thereafter constitute $t_c$ (contacting time). The above-mentioned range of contacting temperatures 85°C-105°C constitute $T_c$ - (contacting temperatures).

Stir at $T_r$ for a period $t_r$: $T_r$ and $t_r$ are as specified below for each example.

(b) Maturation:

Bring temperature to $T_m$ by cooling within 1 hour and maintain this temperature, with or without stirring, for a total duration of the maturation procedure of $t_m$ hours.

(c) Arresting the Reaction; Inorganic Workup:

The procedure (c) of Example XXVII is used. The amounts of water, sodium carbonate and sodium bicarbonate are reduced, in proportion with the scale and stoichiometry of the process.

Starting-Materials:

In each of Examples XXX, XXXI, XXXII and XXXIII:

| | |
|---|---|
| Distilled Water | 115.6 g |
| 50.0% Aqueous NaOH | 156.8 g (1.96 moles NaOH) |
| D,L-Malic Acid | 134.1 g (1.0 mole) |
| $Ca(OH)_2$ | 105.1 g (1.42 moles) |
| Maleic Anhydride | 128.4 g (1.31 moles) |
| Total Weight | 640.0 g |
| Total Weight % of Maleate (as $C_4H_2O_4$) + malate (as $C_4H_4O_5$): | 44.0% |

Temperatures and Times:

In each of Examples XXX, XXXI, XXXII and XXXIII: $T_r$ = 76°C; $t_r$ = 5 hours.

27

Maturation Results:

| Ex. No. | $T_m$ (°C) | $t_m$ (hours) | % ODS* | Composition by HPLC, % | | |
|---|---|---|---|---|---|---|
| | | | | maleate | malate | fumarate |
| XXX | 25 | 168 | 84.4 | 7.8 | 4.2 | 3.7 |
| XXXI | 40 | 120 | 86.0 | 5.3 | 3.7 | 5.0 |
| XXXII | 50 | 72 | 83.0 | 4.3 | 5.6 | 7.2 |
| XXXIII | 60 | 24 | 81.6 | 5.3 | 6.5 | 6.7 |

*2,2'-oxodisuccinate

In the above, at the indicated maturation times, the 2,2'-oxodisuccinate yields are still increasing in the 25°C and 40°C examples, whereas in the 50°C and 60°C examples, the 2,2'-oxodisuccinate yields are near or at their maxima.

EXAMPLE XXXIV

The procedures (a), (b) and (c) of Examples XXX, XXXI, XXXII and XXXIII are carried out with the following modifications: Contacting time $t_c$ is 30 minutes.

Contacting temperatures $T_c$ are in the range from 75°C to 110°C, provided that the reaction mixture is cooled sufficiently so that the temperatures do not exceed 100°C for more than 15 minutes and do not exceed 80°C for more than 30 minutes.

The optional stirring period $t_r$ is not used in this Example ($t_r$ = 0 minutes).

$T_m$ is 40°C

Amounts of Starting-Materials:

| | |
|---|---|
| Distilled Water | 121.9 g |
| 50% Aqueous NaOH | 156.8 g (1.96 moles NaOH) |
| D,L-Malic Acid | 134.1 g (1.0 mole) |
| $Ca(OH)_2$ | 105.1 g (1.42 moles) |
| Maleic Anhydride | 128.4 g (1.31 moles) |
| Total Weight | 646.3 g |

Results:

| Tm °C | Tm hr | % ODS* | % maleate | % malate | % fumarate |
|---|---|---|---|---|---|
| 40° | 48 | 83.3 | 10.8 | 3.5 | 2.3 |
| 40° | 120 | 85.7 | 5.8 | 3.9 | 4.6 |

*2,2'-oxodisuccinate

EXAMPLE XXXV

Equipment: Small-scale version of the equipment used in Example XXVII.

Precontacting of maleate and sodium by preneutralization of maleic acid with sodium carbonate to form disodium maleate *in−situ*:

To a solution at 80°C, made from distilled water (300 grams) and maleic acid (148.5 grams, 1.28 moles) in a weighed, open reaction vessel is added slowly, over 30 minutes, with vigorous stirring, sodium carbonate (116.6 grams, 1.10 moles). Carbon dioxide and a significant fraction of entrained water are allowed to boil off for 30 minutes after completing the addition.

Addition of calcium hydroxide:

Calcium hydroxide (91.8 grams, 1.24 moles) is slowly added to the mixture with stirring. During the next hour, water is evaporated at 70°C to 80°C under a stream of compressed air, so that the total weight of the contents of the reaction vessel is reduced to 504 grams.

Addition of malate and primary 2,2'-oxodisuccinate-forming reaction:

Over a 10 minute period, D,L-malic acid (134.1 grams, 1.0 mole) is added at a steady rate to the vortex of the stirred reaction mixture, held at 100°C. After adding the D,L-malic acid, the temperature of the crude product mixture is reduced over a 10 minute period to 78°C. Stirring is continued at 78°C for a 5 hour period.

Maturation procedure:

Rapidly (in less than 1 hour), the temperature of the crude product mixture is reduced to 50°C. The crude product mixture is held at 50°C for a total duration of the maturation procedure of 72 hours. The crude product mixture now has a 2,2'-oxodisuccinate yield of 82%, as determined by HPLC. The product workup is a scaled-down version of procedure (c) in Example XXVII.

EXAMPLE XXXVI

Equipment: Scaled-down version of the equipment used in Example 1.

Over a 25 minute period at approximately constant temperature of 49°C, maleic anhydride powder (125.4 grams, 128 moles) is added steadily, with stirring, to a mixture preformed by combining:

| | |
|---|---|
| distilled water | 111.5 grams |
| 50% aqueous NaOH | 175.2 grams (2.19 moles) |
| D,L-malic acid | 134.1 grams (1.0 mole) |
| calcium hydroxide | 91.8 grams (1.24 moles) |

The temperature and stirring are maintained for 120 hours, whereupon the crude product mixture which is formed has a HPLC-based 2,2'-oxodisuccinate yield of 82%.

The reaction is arrested without delay, and the crude product mixture is converted to tetrasodium 2,2'-oxodisuccinate by successively (i) treating with sodium carbonate/bicarbonate; (ii) filtering to remove the precipitated calcium carbonate and (iii) conventionally drying the filtrate.

EXAMPLE XXXVII

D,L-malic acid, 134.1 grams, 1.0 mole, is dissolved in 111.5 grams of water in a suitably sized stainless steel reaction vessel equipped with stirring and temperature control means (see Example XXVII). As rapidly as possible, a slurry made by separately mixing sodium hydroxide (175.2 grams, 50% aqueous solution, 2.19 moles NaOH content) and calcium hydroxide (91.8 grams, 1.24 moles) is added while stirring is continued and the temperature is held at 75°C. With the mixture and maleic anhydride both at temperatures in the range 60°C - 70°C, molten maleic anhydride, 125.4 grams, 1.28 moles, is added at a steady rate with continuous stirring over a period of 20 minutes. The temperature and stirring are maintained for a further 10 minutes. The temperature is now permitted to fall to 50°C over a 2 hour period, and the crude product mixture, now almost clear and somewhat more viscous than heretofore, is stirred at 50°C for 94 hours. The temperature is now further quickly reduced to 40°C, and the crude product mixture is stored, unstirred, for 144 hours. The reaction is arrested and the crude product mixture is worked up by sodium carbonate/bicarbonate treatment as in Example XXXVI. At the time of arresting the reaction, the crude product mixture has a HPLC-analyzed organic composition as follows: 2,2'-oxodisuccinate: 88.7%; maleate: 6.7%; malate: 1.8%; fumarate: 2.7%. After workup, the organic analysis is essentially unchanged.

HPLC Analytical Procedures

High Performance Liquid Chromatography (HPLC) analyses herein for maleate, malate, 2,2'-oxodisuccinate and fumarate are readily reproduced using the following conditions, by an analyst familiar with HPLC instrumentation:

| | |
|---|---|
| Column: | Two Supelco LC18 4.6 mm I.D. x 25 cm columns equilibrated with mobile phase for about 10 days at a flow rate of about 0.2 ml/min |
| Mobile Phase: | 0.01-0.04 $\underline{N}$ $H_2SO_4$ in distilled/deionized water. The sulfuric acid concentration is adjusted in dependence of the age of the columns to give separation of each of the organic species analyzed. |
| Flow Rate: | 1 ml/min |
| Pump: | A single Waters 6000A or 510 |
| Injector: | Waters Wisp 710, injection volume 25 ul |
| Detector: | Refractive-Index Detector; Spectra-Physics 6040XR. |
| Integrator: | Waters 730 Data Module |
| Sample Preparation: | For distribution analysis: ca 0.5 g of crude product mixture is removed from the reaction vessel and is quenched by adding to 100 ml 0.1 $\underline{N}$ sulfuric acid; for concentration analysis: weigh a known amount into a 10 ml volumetric flask to give a peak area that falls within the calibration curve, q.s. with 0.1 $\underline{N}$ sulfuric acid. In both cases, the final solution pH is about 2. |

Calculations:

| | |
|---|---|
| Distribution analysis: | Divide peak area for a single species; i.e., maleate, fumarate, 2,2′-oxodisuccinate, malate, by total area for all peaks. This assumes equivalent refractive index response factors for all species; the assumption is proved to be an excellent approximation by concentration analysis. Based on this assumption, the distribution analysis corresponds to the relative weight percentage of the maleate, malate, 2,2′-oxodisuccinate and fumarate analyzed. |
| Concentration analysis: | A direct calibration curve is made for maleic acid, malic acid, and fumaric acid for direct correlation of peak areas to concentration expressed as percentage by weight. The same procedure is used to determine the weight % of 2,2′-oxodisuccinate except that 1,2,3,4-butanetetracarboxylic acid (structurally similar to 2,2′-oxodisuccinate) or chromatographically pure 2,2′-oxodisuccinate is used as the calibration species. Note that use of 1,2,3,4-butanetetracarboxylic acid as a reference material assumes that 2,2'-oxodisuccinate and 1,2,3,4-butanetetracarboxylic acid have similar refractive index response factors. In all cases, a linear regression analysis is used to fit the curves. |

Correspondence between the results obtained for the distribution analysis and the concentration analysis is very good for a given sample. In view of the good correspondence, the tables and text herein report only the simple distribution analysis results.

**Claims**

1. An aqueous process of manufacturing 2,2'-oxodisuccinate by reacting starting-materials comprising:
   I An organic starting-material component comprising maleate;
   II a divalent metal cation component; and
   III an alkali component;
   wherein during said process, said organic starting material component is converted to 2,2'-oxodisuccinate at particular yields, and by-product, especially fumarate, tends to be formed; said aqueous process being comprising the following steps:
   A. selecting starting-materials which comprise:
      I an organic component comprising maleate and preformed malate at a maleate:malate mole ratio in the range from 0.7:1 to 2.0:1;
      II a divalent metal cation component selected from calcium, magnesium and mixtures thereof, at a divalent cation component:organic component mole ratio in the range from 0.1:1 to 0.95:1;
      III an alkali component selected from hydroxide and hydroxide-forming anions; and, additionally,

IV a solubilizing monovalent cation component selected from sodium, potassium and mixtures thereof, at a sodium:potassium mole ratio in the range 1.0:0.0 to 0.9:0.1; and

B. conducting the reaction in a fluid, aqueous alkaline mixture of said starting-materials having the following net concentrations by weight:

no more than 75% water

at least 0.0001% alkali component III, expressed as net excess hydroxide:

at least 1% solubilizing monovalent cation component IV:

said reaction taking place at one or more temperatures in the range from 20°C to 110°C, and

C. arresting the reaction;

characterized by: a total duration of reaction from 12 hours to 400 days; the total time at any temperature above 100°C does not exceed 1.5 hours; the total time at any temperature above 90°C does not exceed 4.5 hours; the total time at any temperature above 80°C does not exceed 13.5 hours; the total time at any temperature above 70°C does not exceed 1.7 days; the total time at any temperature above 60°C does not exceed 5.1 days; the total time at any temperature above 50°C does not exceed 15 days; the total time at any temperature above 40°C does not exceed 46 days; and the total time at any temperature above 30°C does not exceed 137 days;

whereby by-product formation is minimized and a 2,2'-oxodisuccinate yield of 80% or higher is secured.

2. A process according to claim 1 wherein

A. the starting materials comprise:

I maleate and preformed malate at a maleate:malate mole ratio in the range from 0.9:1 to 1.8:1, preferably from 1.05:1 to 1.7:1;

II the diva lent metal cation component at a divalent metal cation component to organic component mole ratio in the range from 0.2:1 to 0.85:1, preferably from 0.25:1 to 0.8:1;

wherein

B. the fluid aqueous alkaline mixture of said starting materials has the following net concentrations by weight: from 25% to 60%, preferably from 30% to 50% water; no more than 2% preferably from 0.01% to 1.5% alkali component III; and

from 3% to 20%, preferably from 3% to 16% solubilizing monovalent cat ion component IV;

wherein the total reaction time is from 12 hours to 240 days, preferably from 12 hours to 40 days and wherein the total time at any temperature above 100°C does not exceed 1 hour, and preferably does not exceed 30 minutes;

the total time at any temperature above 90°C does not exceed 3 hours and preferably does not exceed 1.5 hours; the total time at any temperature above 80°C does not exceed 8 hours and preferably does not exceed 5 hours; the total time at any temperatuire above 70°C does not exceed 1 day and preferably does not exceed 15 hours; the total time at any temperature above 60°C does not exceed 3 days and preferably does not exceed 1.5 days; the total time at any temperature above 50°C does not exceed 9 days and preferably does not exceed 8 days; the total time at any temperature above 40°C does not exceed 27 days and the total time at any temperature above 30°C does not exceed 81 days.

3. A process according to either one of claims 1 and 2 comprising:

A. selecting starting-materials which comprise:

I an organic component comprising maleate and preformed malate at a maleate:malate mole ratio in the range from 1.1:1 to 1.6:1;

II calcium as the divalent metal cation component at a calcium:organic component mole ratio in the range from 0.35:1 to 0.80:1;

III an alkali component selected from hydroxide and hydroxide-forming anions; and, additionally,

IV sodium as the solubilizing monovalent cation component; and

B. conducting the reaction in a fluid, aqueous alkaline mixture of said starting-materials having the following net concentrations by weight: from 30% to 45% water;

from 0.05% to 1% alkali component III, expressed as net excess hydroxide; and

from 3.5% to 12% sodium ion;

said reaction comprising the steps of

(a) an elevated temperature primary reaction stage of duration 10 minutes to 8 hours, at 50°C to 110°C, in which said starting-materials are contacted to form said fluid, aqueous alkaline mixture and reacted to form a crude product mixture which remains fluid, comprising freshly formed 2,2'-oxodisuccinate together with unreacted maleate and malate; followed immediately by

(b) a lower temperature maturation stage of duration 1 day to 30 days, in which the temperature of said crude product mixture of step (a) is reduced in one or more steps whilst retaining fluidity and continuing to react said crude product mixture, for a period sufficient to chemically combine and form 2,2'-oxodisuccinate from said maleate and malate, thereby increasing the overall proportion of 2,2'-oxodisuccinate present in said crude product mixture while achieving control of the rate of formation of fumarate by-product; and

(c) arresting said lower temperature maturation procedure, preferably by treating said crude product mixture with a warm aqueous mixture of sodium carbonate and sodium bicarbonate so as to precipitate calcium carbonate.

4. A process according to Claim 3 wherein said lower temperature maturation stage (b) comprises reducing the temperature of said crude product mixture of step (a) to temperatures in the range from 20°C to 45°C and wherein Component I comprises maleate and preformed malate at a maleate:malate mole ratio in the range from 1.15:1 to 1.40:1; and

Component II comprises calcium ion at a calcium ion:organic component mole ratio in the range from 0.41:1 to 0.76:1; and wherein said process is conducted in a fluid, aqueous alkaline mixture of said starting-materials having the following net concentrations by weight:

   from 35.0% to 41.0% water;

   from 0.10% to 0.91% alkali component III, expressed as net excess hydroxide: and

   from 3.9% to 10.6% solubilizing monovalent cation component IV:

5. A process according to Claim 4 wherein step (a) is carried out at temperatures in the range of from 50°C to 110°C and has a duration of from 10 minutes to 5 hours, provided that in step (a), said temperatures are not in excess of 100°C for times greater than 15 minutes and are not in excess of 80°C for times greater than 30 minutes; and

wherein step (b) comprises reducing the temperature to temperatures in the range of from 20°C to 45°C in a time less than 2 hours and maintaining said temperatures; step (b) having a duration of from 1 day to 21 days in total; provided that in step (b), said temperatures are not in excess of 36°C for times greater than 7 days and are not in excess of 30°C for times greater than 14 days.

6. A process according to any of Claims 1-5 wherein said starting-materials comprise at least one maleate compound selected from maleic anhydride and maleic acid, together with at least one malate compound selected from malic acid and stereoisomers thereof; or wherein said starting-materials comprise at least one sodium-containing compound selected from disodium maleate, disodium malate, sodium carbonate, sodium bicarbonate and sodium hydroxide.

7. A process according to any of Claims 2-6 wherein said starting-materials consist essentially of maleic anhydride, D,L-malic acid, sodium hydroxide and calcium hydroxide and wherein step (a) comprises adding maleic anhydride over a period of 10 to 30 minutes, as portions of solid or as liquid at temperatures above the melting-point but not exceeding 100°C, to a continuously stirred preformed mixture of said D,L-malic acid, calcium hydroxide and sodium hydroxide, the preformed mixture having an initial temperature in the range from 50°C to 85°C, said temperature rising exothermically to a maximum of from 100°C to 110°C during the course of maleic anhydride addition, thereby forming a crude product mixture.

8. A process according to any of claims 2-7 wherein upon ending step (a), said crude product mixture is substantially homogeneous and has an organic composition, as a percentage by weight based on 2,2'-oxodisuccinate plusmaleate plus malate plus fumarate, of:

   2,2'-oxodisuccinate: from 20% to 45%;

   fumarate: from 1% to 2% and

   maleate plus malate: from 53% to 79%;

and wherein said crude product mixture at the end of step (b) has an organic composition comprising, as a percentage by weight based on 2,2'-oxodisuccinate plus maleate plus malate plus fumarate, of:

   2,2'-oxodisuccinate: at least 82%;

   fumarate: from 1.5% to 5.7% and

maleate plus malate: from 7.3% to 16.5%.

9. A process according to any of Claims 2-8 wherein the starting-materials comprise no more than 0.01 moles of fumarate impurity per mole of said maleate plus said preformed malate, and wherein step (a) is ended and step (b) is undertaken at any time corresponding with a net increase in fumarate level, based upon HPLC-analysis of the crude product mixture, in the range from 0.5% to 5%, preferably 0.5% to 2.5%; more preferably wherein step (b) is ended and step (c) is undertaken immediately upon reaching an HPLC-based 2,2'-oxodisuccinate yield of at least 85%.

10. An aqueous process for preparing 2,2'-oxodisuccinate comprising reacting preformed malic acid, sodium hydroxide, a maleate reactant selected from maleic anhydride, maleic acid and mixtures thereof, and a calcium reactant selected from calcium carbonate and mixtures thereof with calcium hydroxide, according to the immediately consecutive steps:

(i) mixing calcium carbonate, water, malic acid and a proportion of said maleate reactant, allowing complete evolution of carbon dioxide and forming an acidic mixture;

(ii) adding sodium hydroxide or a mixture thereof with calcium hydroxide, to the acidic mixture of step (i), forming a sodium cation-containing alkaline mixture;

(iii) in a period of duration 1 hour or less, adding the remainder of said maleate reactant to the stirred sodium cation-containing alkaline mixture of step (ii), at temperatures in the range from 75°C to 110°C, having at the end of the step (iii) addition a net hydroxide excess $M_{OH}$;

(iv) in a period of duration 1 hour or less, cooling the mixture formed in step (iii) to a temperature in the range from 35°C to 45°C;

(v) at said temperature in the range from 35°C to 45°C, continuing to react the mixture of step (iv); the duration of step (v) being from 48 hours to 240 hours, whereby a crude product having a HPLC yield of at least 80% 2,2'-oxodisuccinate is secured;

(vi) diluting the product of step (v) with water and precipitating calcium carbonate therefrom; thereby arresting the step (iv) reaction and depleting the level of calcium;

provided that in steps (iii) and (iv) together;

- the total time at any temperature above 100°C does not exceed 15 minutes;
- the total time at any temperature above 90°C does not exceed 30 minutes;
- the total time at any temperature above 80°C does not exceed 2 hours;
- the total time at any temperature above 70°C does not exceed 6 hours;
- the total time at any temperature above 65°C does not exceed 12 hours; and

provided that for each mole of preformed malic acid reacted, the total molar amount of maleate reactant, is 1.1 to 1.6 moles; the total molar amount of calcium reactant, is from 0.9 to 1.65 moles; the total molar amount of sodium hydroxide, is from 0.92 to 3.7 moles; the net hydroxide excess in step (iii), $M_{OH}$, is from 0.02 to 0.3 moles; and further provided that for each mole of preformed malic acid reacted, the total net amount of water added in steps (i), (ii) and (iii) together, allowing for evaporation losses, is no less than 189 grams and no more than 282 grams.

11. A process according to Claim 10 comprising the additional steps (vii) filtering the mixture of step (vi) to secure a filter-cake and (viii) using the filter-cake of step (vii) as recycled source of calcium carbonate in step (i).

12. A process according to anyone of Claims 1-11 wherein the preformed malate is made by a process comprising at least one step of maintaining, in a reaction vessel, preferably of sealed construction and having a pressure in the range from 0.21 MPa to 1.03 MPa at a temperature of 120°C or above, more preferably 130°C to 220°C, for a time of at least 10 minutes, more preferably 30 minutes to 6 hours, an aqueous reaction mixture comprising:

I maleate, or mixtures thereof with one or more of 2,2'-oxodisuccinate, fumarate and malate;

II divalent cations selected from calcium, magnesium and mixtures thereof; and

III sufficient hydroxide excess to render said reaction mixture alkaline;

whereby said malate is secured in yields up to and exceeding 90%.

13. A process according to Claim 12 wherein component I is used in the form of maleic anhydride or maleic acid, and components II and III ire simultaneously provided in the form of calcium hydroxide.

14. A process according to Claim 12 or 13 using a calcium/maleate mole ratio of from 1.3:1 to 1.7:1 and a water content of said reaction mixture in the range from 45% to 73% by weight.

15. An aqueous alkaline process according to Claim 12 for securing malate in a solid, alkaline, calcium or mixed calcium/magnesium salt form by the steps comprising, in sequence:

    i. reacting maleic anhydride or maleic acid and calcium carbonate to form a mixture;

    ii. permitting carbon dioxide to evolve;

    iii. rendering said mixture alkaline using calcium hydroxide or mixture thereof with magnesium hydroxide; all provided that said reactants are used at a (calcium + magnesium):maleate molar ratio in the range from greater than 1.3:1 to less than 1.7:1, a calcium:magnesium mole ratio in the range from 1.00:0.00 to 0.80:0.20, a water content in the range from 45% to 73% and an excess of hydroxide of from 0.6 moles to 1.4 moles per mole of maleate used; and

    iv. reacting said mixture in a sealed vessel at temperatures in the range from 140°C to 190°C for a period of from 0.5 to 6 hours.


**Patentansprüche**

1. Wäßriges Verfahren zur Herstellung von 2,2'-Oxodisuccinat durch Umsetzung von Ausgangsmaterialien, welche

    I eine Maleat umfassende organische Ausgangsmaterialkomponente;

    II eine zweiwertige Metallkationenkomponente; und

    III eine Alkalikomponente;

umfassen, wobei während des Verfahrens die organische Ausgangsmaterialkomponente in bestimmten Ausbeuten in 2,2'-Oxodisuccinat umgewandelt wird und die Neigung zur Bildung von Nebenprodukt, insbesondere Fumarat, besteht; wobei das wäßrige Verfahren die folgenden Schritte umfaßt:

    A. Auswählen von Ausgangsmaterialien, welche

        I eine Maleat und vorgebildetes Malat bei einem Maleat:Malat-Molverhältnis im Bereich von 0,7:1 bis 2,0:1 umfassende organische Komponente;

        II eine aus Calcium, Magnesium und Mischungen davon gewählte, zweiwertige Metallkationenkomponente bei einem zweiwertige Kationenkomponente:organische Komponente-Molverhältnis im Bereich von 0,1:1 bis 0,95:1;

        III eine aus Hydroxid und hydroxidbildenden Anionen gewählte Alkalikomponente; und weiterhin

        IV eine aus Natrium, Kalium und Mischungen davon bei einem Natrium:Kalium-Molverhältnis im Bereich von 1,0:0,0 bis 0,9:0,1 gewählte, solubilisierende einwertige Kationenkomponente umfassen; und

    B. Durchfuhren der Umsetzung in einer fluiden, wäßrigen alkalischen Mischung der Ausgangsmaterialien mit den folgenden Netto-Gewichtskonzentrationen:

    nicht mehr als 75 % Wasser;

    mindestens 0,0001 % Alkalikomponente III, angegeben als Netto-Hydroxidüberschuß;

    mindestens 1 % solubilisierende einwertige Kationenkomponente IV; wobei die Umsetzung bei einer oder mehreren Temperaturen im Bereich von 20°C bis 110°C stattfindet, und

    C. Hemmen der Umsetzung;

    **dadurch gekennzeichnet**, daß die Gesamtzeit der Umsetzung 12 Stunden bis 400 Tage beträgt; die Gesamtzeit bei einer Temperatur oberhalb 100°C 1,5 Stunden nicht überschreitet; die Gesamtzeit bei einer Temperatur oberhalb 90°C 4,5 Stunden nicht überschreitet; die Gesamtzeit bei einer Temperatur oberhalb 80°C 13,5 Stunden nicht überschreitet; die Gesamtzeit bei einer Temperatur oberhalb 70°C 1,7 Tage nicht überschreitet; die Gesamtzeit bei einer Temperatur oberhalb 60°C 5,1 Tage nicht überschreitet; die Gesamtzeit bei einer Temperatur oberhalb 50°C 15 Tage nicht überschreitet; die Gesamtzeit bei einer Temperatur oberhalb 40°C 46 Tage nicht überschreitet; und die Gesamtzeit bei einer Temperatur oberhalb 30°C 137 Tage nicht überschreitet; wodurch die Nebenproduktbildung minimiert wird und eine 2,2'-Oxodisuccinatausbeute von 80 % oder mehr sichergestellt wird.

2. Verfahren nach Anspruch 1, wobei

    A. die Ausgangsmaterialien

        I Maleat und vorgebildetes Malat bei einem Maleat:Malat-Molverhältnis im Bereich von 0,9:1 bis 1,8:1, vorzugsweise 1,05:1 bis 1,7:1;

        II die zweiwertige Metallkationenkomponente bei einem zweiwertige Metallkationenkomponente-zu-organische Komponente-Molverhältnis im Bereich von 0,2:1 bis 0,85:1, vorzugsweise 0,25:1 bis 0,8:1 umfassen;

    wobei

B. die fluide wäßrige alkalische Mischung der Ausgangsmaterialien die folgenden Netto-Gewichtskonzentrationen aufweist: 25 bis 60%, vorzugsweise 30 bis 50 % Wasser; nicht mehr als 2 %; vorzugsweise 0,01 bis 1,5% Alkalikomponente III; und 3 bis 20 %, vorzugsweise 3 bis 16 % solubilisierende einwertige Kationenkomponente IV; wobei die Gesamtreaktionszeit 12 Stunden bis 240 Tage, vorzugsweise 12 Stunden bis 40 Tage beträgt und wobei die Gesamtzeit bei einer Temperatur oberhalb 100°C 1 Stunde nicht überschreitet und vorzugsweise 30 Minuten nicht überschreitet; die Gesamtzeit bei einer Temperatur oberhalb 90°C 3 Stunden nicht überschreitet und vorzugsweise 1,5 Stunden nicht überschreitet; die Gesamtzeit bei einer Temperatur oberhalb 80°C 8 Stunden nicht überschreitet und vorzugsweise 5 Stunden nicht überschreitet; die Gesamtzeit bei einer Temperatur oberhalb 70°C 1 Tag nicht überschreitet und vorzugsweise 15 Stunden nicht überschreitet; die Gesamtzeit bei einer Temperatur oberhalb 60°C 3 Tage nicht überschreitet und vorzugsweise 1,5 Tage nicht überschreitet; die Gesamtzeit bei einer Temperatur oberhalb 50°C 9 Tage nicht überschreitet und vorzugsweise 8 Tage nicht überschreitet; die Gesamtzeit bei einer Temperatur oberhalb 40°C 27 Tage nicht überschreitet und die Gesamtzeit bei einer Temperatur oberhalb 30°C 81 Tage nicht überschreitet.

3. Verfahren nach Anspruch 1 oder 2, umfassend

A. Auswählen von Ausgangsmaterialien, welche

I eine Maleat und vorgebildetes Malat bei einem Maleat:Malat-Molverhältnis im Bereich von 1,1:1 bis 1,6:1 umfassende organische Komponente;

II Calcium als zweiwertige Metallkationenkomponente bei einem Calcium:organische Komponente-Molverhältnis im Bereich von 0,35:1 bis 0,80:1;

III eine aus Hydroxid und hydroxidbildenden Anionen gewählte Alkalikomponente; und weiterhin

IV Natrium als solubilisierende einwertige Kationenkomponente umfassen; und

B. Durchführen der Umsetzung in einer fluiden, wäßrigen alkalischen Mischung der Ausgangsmaterialien mit den folgenden Netto-Gewichtskonzentrationen:

30 bis 45 % Wasser;

0,05 bis 1 % Alkalikomponente III, angegeben als Netto-Hydroxidüberschuß; und

3,5 bis 12 % Natriumion;

wobei die Umsetzung die Schritte

(a) einer primären Reaktionsstufe bei erhöhter Temperatur einer Dauer von 10 Minuten bis 8 Stunden bei 50°C bis 110°C, in welcher die Ausgangsmaterialien zu Bildung der fluiden, wäßrigen alkalischen Mischung kontaktiert und zur Bildung einer fluid bleibenden Rohproduktmischung, welche frisch gebildetes 2,2'-Oxodisuccinat zusammen mit nichtreagiertem Maleat und Malat umfaßt, umgesetzt werden; unmittelbar gefolgt von

(b) einer Alterungsstufe bei niedrigerer Temperatur einer Dauer von 1 Tag bis 30 Tagen, in welcher die Temperatur der Rohproduktmischung aus Schritt (a) in einem oder mehreren Schritten reduziert wird, während Fluidität beibehalten wird und die Umsetzung der Rohproduktmischung während eines ausreichenden Zeitraums weitergeführt wird, um aus dem Maleat und Malat chemisch 2,2'-Oxodisuccinat zu kombinieren und zu bilden, wodurch der Gesamtanteil von in der Rohproduktmischung vorliegendem 2,2'-Oxodisuccinat erhöht wird, während eine Regulierung der Bildungsrate von Fumarat-Nebenprodukt erzielt wird; und

(c) Hemmen der Alterungsprozedur bei niedrigerer Temperatur, vorzugsweise durch Behandeln der Rohproduktmischung mit einer warmen wäßrigen Mischung aus Natriumcarbonat und Natriumbicarbonat, um so Calciumcarbonat auszufällen, umfaßt.

4. Verfahren nach Anspruch 3, wobei die Alterungsstufe (b) bei niedrigerer Temperatur die Verringerung der Temperatur der Rohproduktmischung aus Schritt (a) auf Temperaturen im Bereich von 20 bis 45°C umfaßt und wobei Komponente I Maleat und vorgebildetes Malat in einem Maleat:Malat-Molverhältnis von 1,15:1 bis 1,40:1 umfaßt; und

Komponente II Calciumionen bei einem Calciumionen:organische Komponente-Molverhältnis im Bereich von 0,41:1 bis 0,76:1 umfaßt; und wobei das Verfahren in einer fluiden, wäßrigen alkalischen Mischung der Ausgangsmaterialien mit den folgenden Netto-Gewichtskonzentration durchgeführt wird:

35,0 bis 41,0% Wasser;

0,10 bis 0,91 % Alkalikomponente III, angegeben als Netto-Hydroxidüberschuß; und

3,9 bis 10,6 % solubilisierende einwertige Kationenkomponente IV.

**5.** Verfahren nach Anspruch 4, wobei Schritt (a) bei einer Temperatur im Bereich von 50 bis 110°C durchgeführt wird und eine Dauer von 10 Minuten bis 5 Stunden aufweist, mit der Maßgabe, daß in Schritt (a) die Temperaturen nicht über 100°C während Zeiträumen von mehr als 15 Minuten und nicht mehr als 80°C während Zeiträumen von mehr als 30 Minuten betragen; und wobei Schritt (b) die Verringerung der Temperatur auf Temperaturen im Bereich von 20 bis 45°C In einer Zelt von weniger als 2 Stunden und das Halten dieser Temeperaturen umfaßt; wobei Schritt (b) eine Dauer von insgesamt 1 Tag bis 21 Tage besitzt; mit der Maßgabe, daß in Schritt (b) die Temperaturen nicht über 36°C während Zeiträumen von mehr als 7 Tagen und nicht über 30°C während Zeiträumen von mehr als 14 Tagen betragen.

**6.** Verfahren nach mindestens einem der Ansprüche 1 bis 5, wobei die Ausgangsmaterialien mindestens eine aus Maleinsäureanhydrid und Maleinsäure gewählte Maleatverbindung, zusammen mit mindestens einer aus Äpfelsäure und Stereoisomeren davon gewählten Malatverbindung umfassen; oder wobei die Ausgangsmaterialien mindestens eine aus Dinatriummaleat, Dinatriummalat, Natriumcarbonat, Natriumbicarbonat und Natriumhydroxid gewählte, natriumhaltige Verbindung umfassen.

**7.** Verfahren nach mindestens einem der Ansprüche 2 bis 6, wobei die Ausgangsmaterialien im wesentlichen aus Maleinsäureanhydrid, D,L-Äpfelsäure, Natriumhydroxid und Calciumhydroxid bestehen und wobei Schritt (a) die Zugabe von Maleinsäureanhydrid über einen Zeitraum von 10 bis 30 Minuten als Portionen eines Feststoffs oder als Flüssigkeit bei Temperaturen oberhalb des Schmelzpunktes, jedoch nicht über 100°C, zu einer kontinuierlich gerührten, vorgebildeten Mischung aus der D,L-Äpfelsäure, Calciumyhdroxid und Natriumhydroxid umfaßt, wobei die vorgebildete Mischung eine Ausgangstemperatur im Bereich von 50 bis 85°C aufweist, die Temperatur exotherm auf ein Maximum von 100 bis 110°C während des Verlaufs der Maleinsäureanhydridzugabe ansteigt, wodurch eine Rohproduktmischung gebildet wird.

**8.** Verfahren nach mindestens einem der Ansprüche 2 bis 7, wobei nach Beendigung des Schritts (a) die Rohproduktmischung im wesentlichen homogen ist und eine organische Zusammensetzung, angegeben als Gew.-% auf Basis von 2,2'-Oxodisuccinat plus Maleat plus Malat plus Fumarat von
2,2'-Oxodisuccinat: 20 bis 45 %;
Fumarat: 1 bis 2 % und
Maleat plus Malat: 53 bis 79 % aufweist;
und wobei die Rohproduktmischung am Ende des Schritts (b) eine organische Zusammensetzung aufweist, welche, angegeben als Gew.-% auf Basis von 2,2'-Oxodisuccinat plus Maleat plus Malat plus Fumarat von:
2,2'-Oxodisuccinat: mindestens 82 %;
Fumarat: 1,5 bis 5,7 % und
Maleat plus Malat: 7,3 bis 16,5 % aufweist.

**9.** Verfahren nach mindestens einem der Ansprüche 2 bis 8, wobei die Ausgangsmaterialien nicht mehr als 0,01 Mol Fumarat Verunreinigung pro Mol des Maleats plus dem vorgebildeten Malat umfassen, und wobei Schritt (a) beendet und Schritt (b) zu irgendeinem Zeitpunkt entsprechend einer Nettozunahme des Fumaratgehaltes, basierend auf HPLC-Analyse der Rohproduktmischung, im Bereich von 0,5 bis 5 %, vorzugsweise 0,5 bis 2,5 %, begonnen wird; wobei weiter vorzugsweise Schritt (b) beendet und Schritt (c) unmittelbar begonnen wird, nachdem eine 2,2'-Oxodisuccinat-Ausbeute auf HPLC-Basis von mindestens 85 % erreicht ist.

**10.** Wäßriges Verfahren zur Herstellung von 2,2'-Oxodisuccinat, umfassend die Umsetzung von vorgebildeter Äpfelsäure, Natriumyhdroxid, einem aus Maleinsäureanhydrid, Maleinsäure und Mischungen davon gewählten Maleat-Reaktanten, und einem aus Calciumcarbonat und Mischungen davon mit Calciumhydroxid gewählten Calciumreaktanten gemäß den unmittelbar aufeinanderfolgenden Schritten:
(i) Mischen von Calciumcarbonat, Wasser, Äpfelsäure und eines Teils des Maleat-Reaktanten, vollständiges Entwickelnlassen von Kohlendioxid und Bilden einer sauren Mischung;
(ii) Zugeben von Natriumhydroxid oder einer Mischung davon mit Calciumhydroxid zu der saueren Mischung aus Schritt (i), Bilden einer Natriumkation enthaltenden alkalischen Mischung;
(iii) während eines Zeitraums von 1 Stunde oder weniger, Zugeben des restlichen Maleatreaktanten zu der gerührten, Natriumkation enthaltenden alkalischen Mischung aus Schritt (ii) bei Temperaturen im Bereich von 75 bis 110°C, wobei am Ende der Schritt (iii)-Zugabe ein Netto-Hydroxidüberschuß MOH vorliegt;

(iv) während eines Zeitraums von 1 Stunde oder weniger, Kühlen der in Schritt (iii) gebildeten Mischung auf eine Temperatur im Bereich von 35 bis 45°C;

(v) bei der Temperatur im Bereich von 35 bis 45°C, Weiterführen der Umsetzung der Mischung aus Schritt (iv); wobei die Dauer des Schritts (v) 48 Stunden bis 240 Stunden beträgt, wodurch ein Rohprodukt mit einer HPLC-Ausbeute von mindestens 80 % an 2,2'-Oxodisuccinat sichergestellt wird;

(vi) Verdünnen des Produkts aus Schritt (v) mit Wasser und Ausfällen von Calciumcarbonat daraus; wodurch die Umsetzung des Schritts (iv) gehemmt wird und Abreichern des Calciumgehaltes;

mit der Maßgabe, daß zusammen in den Schritten (iii) und (iv)
- die Gesamtzeit bei einer Temperatur oberhalb 100°C 15 Minuten nicht überschreitet;
- die Gesamtzeit bei einer Temperatur oberhalb 90°C 30 Minuten nicht überschreitet;
- die Gesamtzeit bei einer Temperatur oberhalb 80°C 2 Stunden nicht überschreitet;
- die Gesamtzeit bei einer Temperatur oberhalb 70°C 6 Stunden nicht überschreitet;
- die Gesamtzeit bei einer Temperatur oberhalb 65°C 12 Stunden nicht überschreitet; und

mit der Maßgabe, daß je Mol umgesetzter, vorgebildeter Äpfelsäure die Gesamtmolmenge an Maleatreaktant 1,1 bis 1,6 Mole beträgt; die Gesamtmolmenge an Calciumreaktant 0,9 bis 1,65 Mole beträgt; die Gesamtmolmenge an Natriumhydroxid 0,92 bis 3,7 Mole beträgt; der Netto-Hydroxidüberschuß in Schritt (iii), MOH, 0,02 bis 0,3 Mole beträgt; und mit der weiteren Maßgabe, daß für jedes Mol umgesetzter, vorgebildeter Äpfelsäure die Gesamtnettomenge an zusammen in den Schritten (i), (ii) und (iii) zugesetztem Wasser, um Verdampfungsverluste zuzulassen, nicht weniger als 189 g und nicht mehr als 282 g beträgt.

11. Verfahren nach Anspruch 10, umfassend die weiteren Schritte (vii) Filtern der Mischung aus Schritt (vi), um einen Filterkuchen sicherzustellen und (viii) Verwenden des Filterkuchens aus Schritt (vii) als Wiederverwertungsquelle für Calciumcarbonat in Schritt (i).

12. Verfahren nach mindestens einem der Ansprüche 1 - 11, wobei das vorgebildete Malat durch ein Verfahren hergestellt wird, welches mindestens einen Schritt des Haltens in einem Reaktionsbehälter, vorzugsweise in Form einer verschlossenen Konstruktion und bei einem Druck im Bereich von 0,21 MPa bis 1,03 MPa bei einer Temperatur von 120°C oder darüber, weiter vorzugsweise 130 bis 220°C, während eines Zeitraums von mindestens 10 Minuten, weiter vorzugsweise 30 Minuten bis 6 Stunden, einer wäßrigen Reaktionsmischung umfaßt, welche

I Maleat oder Mischungen davon mit einem oder mehreren aus 2,2'-Oxodisuccinat, Fumarat und Malat;

II aus Calcium, Magnesium und Mischungen davon gewählte zweiwertige Kationen; und

III ausreichend Hydroxidüberschuß, um die Reaktionsmischung alkalisch zu machen; umfaßt;

wodurch das Malat in Ausbeuten bis zu und von mehr als 90 % sichergestellt wird.

13. Verfahren nach Anspruch 12, wobei Komponente I in Form von Maleinsäureanhydrid oder Maleinsäure verwendet wird und die Komponenten II und III gleichzeitig in Form von Calciumhydroxid vorgesehen werden.

14. Verfahren nach Anspruch 12 oder 13, umfassend die Verwendung eines Calcium/Maleat-Molverhältnisses von 1,3:1 bis 1,7:1 und einen Wassergehalt der Reaktionsmischung im Bereich von 45 bis 73 Gew.-%.

15. Wäßriges alkalisches Verfahren nach Anspruch 12 zur Sicherung von Malat in einer festen, alkalischen, Calcium- oder Calcium/Magnesium-Mischsalzform, durch die Schritte, welche der Reihe nach:

i. Umsetzen von Maleinsäureanhydrid oder Maleinsäure und Calciumcarbonat zur Bildung einer Mischung;

ii. Entwickelnlassen von Kohlendioxid;

iii. Alkalischmachen der Mischung unter Verwendung von Calciumhydroxid oder eine Mischung davon mit Magnesiumhydroxid; jeweils mit der Maßgabe, daß die Reaktanten bei einem (Calcium + Magnesium):Maleat-Molverhältnis im Bereich mehr als 1,3:1 bis weniger als 1,7:1, einem Calcium:Magnesium-Molverhältnis im Bereich von 1,00:0,00 bis 0,80:0,20, einem Wassergehalt im Bereich von 45 bis 73 % und einem Hydroxidüberschuß von 0,6 Molen bis 1,4 Molen pro Mol verwendetem Maleat eingesetzt werden; und

iv. Umsetzen der Mischung in einem geschlossenen Behälter bei Temperaturen im Bereich von 140 bis 190°C während eines Zeitraums von 0,5 bis 6 Stunden umfassen.

**Revendications**

1. Procédé aqueux pour fabriquer un 2,2'-oxodisuccinate en faisant réagir des substances de départ comprenant:

I un composant de départ organique comprenant un maléate;

II un composant de type cation de métal divalent; et

III un composant de type alcali; dans lequel, pendant ledit procédé, ledit composant de départ organique est transformé en 2,2'-oxodisuccinate à des rendements particuliers, et un sous-produit, spécialement du fumarate, tend à se former; ledit procédé aqueux comprenant les étapes suivantes:

A. on choisit des substances de départ qui comprennent:

I un composant organique comprenant du maléate et du malate préformé avec un rapport molaire maléate:malate dans l'intervalle de 0,7:1 à 2,0:1;

II un composant de type cation de métal divalent choisi parmi le calcium, le magnésium et des mélanges de ceux-ci, à un rapport molaire composant de type cation divalent: composant organique dans l'intervalle de 0,1:1 à 0,95:1;

III un composant de type alcali choisi parmi des anions hydroxydes et formateurs d'hydroxydes; et de plus,

IV un composant de type cation monovalent de solubilisation choisi parmi le sodium, le potassium et des mélanges de ceux-ci, à un rapport molaire sodium:potassium dans l'intervalle de 1,0:0,0 à 0,9:0,1; et

B. on conduit la réaction dans un mélange alcalin, aqueux, fluide, desdites substances de départ ayant les concentrations nettes suivantes en poids:

pas plus de 75% d'eau,

au moins 0,0001% de composant de type alcali III, exprimé en excès net d'hydroxyde,

au moins 1% de composant de type cation monovalent de solubilisation IV,

ladite réaction ayant lieu à une ou plusieurs températures dans l'intervalle de 20°C à 110°C, et

C. on arrête la réaction; caractérisé par une durée totale de réaction allant de 12 heures à 400 jours; la durée totale à une température quelconque au-dessus de 100°C n'excédant pas 1,5 heure; la durée totale à une température quelconque au-dessus de 90°C n'excédant pas 4,5 heures; la durée totale à une température quelconque au-dessus de 80°C n'excédant pas 13,5 heures; la durée totale à une température quelconque au-dessus de 70°C n'excédant pas 1,7 jour; la durée totale à une température quelconque au-dessus de 60°C n'excédant pas 5,1 jours; la durée totale à une température quelconque au-dessus de 50°C n'excédant pas 15 jours; la durée totale à une température quelconque au-dessus de 40°C n'excédant pas 46 jours; et la durée totale à une température quelconque au-dessus de 30°C n'excédant pas 137 jours;

grâce à quoi la formation de sous-produit est minimisée et un rendement de 80% ou plus de 2,2'-oxodisuccinate est assuré.

2. Procédé selon la revendication 1, dans lequel

A. les substances de départ comprennent:

I du maléate et du malate préformé avec un rapport molaire maléate:malate dans l'intervalle de 0,9:1 à 1,8:1, de préférence de 1,05:1 à 1,7:1;

II le composant de type cation de métal divalent avec un rapport molaire composant de type cation de métal divalent à composant organique dans l'intervalle allant de 0,2:1 à 0,85:1, de préférence de 0,25:1 à 0,8:1;

dans lequel

B. le mélange alcalin aqueux fluide desdites substances de départ possède les concentrations nettes suivantes en poids: de 25% à 60%, de préférence de 30% à 50% d'eau; pas plus de 2%, de préférence de 0,01% à 1,5% de composant de type alcali III; et de 3% à 20%, de préférence de 3% à 16% de composant de type cation monovalent de solubilisation IV;

dans lequel la durée totale de réaction va de 12 heures à 240 jours, de préférence, de 12 heures à 40 jours et dans lequel la durée totale à une température quelconque au-dessus de 100°C n'excède pas 1 heure et, de préférence, n'excède pas 30 minutes;

la durée totale à une température quelconque au-dessus de 90°C n'excède pas 3 heures et de préférence n'excède pas 1,5 heure; la durée totale à une température quelconque au-dessus de 80°C n'excède pas 8 heures et de préférence n'excède pas 5 heures; la durée totale à une température quelconque au-dessus de 70°C n'excède pas 1 jour et de préférence n'excède pas 15 heures; la durée totale à une température quelconque au-dessus de 60°C n'excède pas 3 jours et de préférence n'excède pas 1,5 jour; la durée totale à une température quelconque au-dessus de 50°C n'excède pas 9 jours et de préférence n'excède pas 8 jours; la durée totale à une température quelconque au-dessus de 40°C n'excède pas 27 jours et la durée totale à une température quelconque au-dessus de 30°C n'excède pas 81 jours.

3.  Procédé selon l'une ou l'autre des revendications 1 et 2 comprenant les étapes consistant:
   A. à choisir des substances de départ qui comprennent:
      I un composant organique comprenant du maléate et du malate préformé avec un rapport molaire maléate:malate dans l'intervalle allant de 1,1:1 à 1,6:1;
      II du calcium comme composant de type cation de métal divalent avec un rapport molaire calcium:composant organique dans l'intervalle allant de 0,35:1 à 0,80:1;
      III un composant de type alcali choisi parmi des anions hydroxydes et formateurs d'hydroxydes; et, de plus,
      IV du sodium comme composant de type cation monovalent de solubilisation; et
   B. à conduire la réaction dans un mélange alcalin, aqueux, fluide, desdites substances de départ ayant les concentrations nettes suivantes en poids:
   de 30% à 45% d'eau;
   de 0,05% à 1% de composant de type alcali III, exprimé en excès net d'hydroxyde; et de 3,5% à 12% d'ions sodium;
   ladite réaction comprenant les étapes
      (a) d'une phase de réaction primaire à température élevée durant une période allant de 10 minutes à 8 heures, à une température allant de 50°C à 110°C, dans laquelle on met en contact lesdites substances de départ pour former ledit mélange alcalin, aqueux, fluide, et on les fait réagir pour former un mélange de produit brut qui reste fluide, comprenant du 2,2'-oxodisuccinate nouvellement formé ainsi que du maléate et du malate n'ayant pas réagi; immédiatement suivie
      b) d'une phase de maturation à température plus faible, d'une durée de 1 jour à 30 jours, dans laquelle la température dudit mélange de produit brut de la phase (a) est réduite en une ou plusieurs étapes tout en maintenant la fluidité et en continuant à faire réagir ledit mélange de produit brut, pendant une période suffisante pour combiner et former chimiquement du 2,2'-oxodisuccinate à partir desdits maléate et malate, en augmentant ainsi la proportion globale de 2,2'-oxodisuccinate présent dans ledit mélange de produit brut, tout en réalisant le contrôle du taux de formation du sous-produit de type fumarate; et
      (c) d'une procédure d'arrêt de ladite maturation à température plus faible, de préférence en traitant ledit mélange de produit brut, avec un mélange aqueux, chaud, de carbonate de sodium et de bicarbonate de sodium de façon à précipiter le carbonate de calcium.

4.  Procédé selon la revendication 3, dans lequel ladite phase de maturation à température plus faible (b) comprend la réduction de la température dudit mélange de produit brut de l'étape (a) à des températures dans l'intervalle de 20°C à 45°C et dans lequel le composant I comprend du maléate et du malate préformé avec un rapport molaire maléate:malate dans l'intervalle allant de 1,15:1 à 1,40:1; et
   le composé II comprend de l'ion calcium avec un rapport molaire ion calcium:composant organique dans l'intervalle allant de 0,41:1 à 0,76:1 et dans lequel, ledit procédé est conduit dans un mélange alcalin, aqueux, fluide, desdites substances de départ ayant les concentrations nettes suivantes en poids:
   de 35,0% à 41,0% d'eau;
   de 0,10% à 0,91% de composant de type alcali III, exprimé en excès net d'hydroxyde;
   de 3,9% à 10,6% de composant de type cation monovalent de solubilisation IV.

5.  Procédé selon la revendication 4, dans lequel l'étape (a) est effectuée à des températures dans l'intervalle allant de 50°C à 110°C et dure de 10 minutes à 5 heures, à condition que dans l'étape (a), lesdites températures ne dépassent pas 100°C pendant des durées supérieures à 15 minutes et ne dépassent pas 80°C pendant des durées supérieures à 30 minutes; et dans lequel l'étape (b) comporte la réduction de la température à des températures dans l'intervalle allant de 20°C à 45°C en un temps inférieur à 2 heures et le maintien desdites températures; l'étape (b) ayant au total une durée de 1 jour à 21 jours; à condition que dans l'étape (b), lesdites températures ne dépassent pas 36°C pendant des durées supérieures à 7 jours et ne dépassent pas 30°C pendant des durées supérieures à 14 jours.

6. Procédé selon l'une quelconque des revendications 1 à 5, dans lequel lesdites substances de départ comprennent au moins un composé maléate choisi parmi l'anhydride maléique et l'acide maléique, avec au moins un composé malate choisi parmi l'acide malique et ses stéréoisomères; ou dans lequel lesdites substances de départ comprennent au moins un composé contenant du sodium, choisi parmi le maléate disodique, le malate disodique, le carbonate de sodium, le bicarbonate de sodium et l'hydroxyde de sodium.

7. Procédé selon l'une quelconque des revendications 2 à 6, dans lequel lesdites substances de départ sont constituées essentiellement d'anhydride maléique, d'acide D,L-malique, d'hydroxyde de sodium et d'hydroxyde de calcium, et dans lequel l'étape (a) comprend l'addition d'anhydride maléique sur une période de 10 à 30 minutes, sous forme de fractions de matières solides ou sous forme liquide à des températures supérieures au point de fusion mais ne dépassant pas 100°C, à un mélange préformé continuellement agité desdits acide D,L-malique, hydroxyde de calcium et hydroxyde de sodium, le mélange préformé ayant une température initiale dans l'intervalle allant de 50°C à 85°C, ladite température s'élevant exothermiquement jusqu'à un maximum de 100°C à 110°C au cours de l'addition d'anhydride maléique, en formant ainsi un mélange de produit brut.

8. Procédé selon l'une quelconque des revendications 2 à 7, dans lequel après achèvement de l'étape (a), ledit mélange de produit brut est sensiblement homogène et possède une composition organique, en pourcentage en poids par rapport au 2,2'-oxodisuccinate plus le maléate plus le malate plus le fumarate, de:
   2,2'-oxodisuccinate: de 20% à 45%;
   fumarate: de 1% à 2%; et
   maléate plus malate: de 53% à 79%;
et dans lequel ledit mélange de produit brut possède à la fin de l'étape (b) une composition organique comprenant, en pourcentage en poids par rapport au 2,2'-oxodisuccinate plus le maléate plus le malate plus le fumarate, de:
   2,2'-oxodisuccinate: au moins 82%;
   fumarate: de 1,5% à 5,7%; et
   maléate plus malate: de 7,3% à 16,5%.

9. Procédé selon l'une quelconque des revendications 2 à 8, dans lequel les substances de départ ne comprennent pas plus de 0,01 mole d'impureté de fumarate par mole dudit maléate plus dudit malate préformé, et dans lequel l'étape (a) est achevée et l'étape (b) est entreprise à tout moment correspondant à un accroissement net du taux de fumarate, sur la base de l'analyse par HPLC du mélange de produit brut, dans l'intervalle allant de 0,5% à 5%, de préférence de 0,5% à 2,5%; de manière encore préférée dans lequel l'étape (b) est achevée et l'étape (c) est entreprise immédiatement lorsqu'on atteint un rendement de 2,2'-oxodisuccinate sur la base de la HPLC, d'au moins 85%.

10. Procédé aqueux pour préparer le 2,2-oxodisuccinate comprenant la mise en réaction de l'acide malique préformé, de l'hydroxyde de sodium, d'un réactif de type maléate choisi parmi l'anhydride maléique, l'acide maléique et leurs mélanges, et d'un réactif de type calcium choisi parmi le carbonate de calcium et des mélanges de celui-ci avec l'hydroxyde de calcium selon les étapes immédiatement consécutives:
   (i) on mélange du carbonate de calcium, de l'eau, de l'acide malique et une proportion dudit réactif de type maléate, en permettant un dégagement complet du dioxyde de carbone et en formant un mélange acide;
   (ii) on ajoute de l'hydroxyde de sodium ou un mélange de ce dernier avec de l'hydroxyde de calcium, au mélange acide de l'étape (i), en formant un mélange alcalin contenant le cation sodium;
   (iii) pendant une période de temps de 1 heure ou moins, on ajoute le reste dudit réactif de type maléate au mélange alcalin agité contenant le cation sodium de l'étape (ii), à des températures dans l'intervalle allant de 75°C à 110°C, en ayant à la fin de l'étape d'addition (iii) un excès net d'hydroxyde $M_{OH}$;

(iv) pendant une période durant 1 heure ou moins, on refroidit le mélange formé dans l'étape (iii) jusqu'à une température dans l'intervalle allant de 35°C à 45°C;

(v) à ladite température dans l'intervalle de 35°C à 45°C, on continue à faire réagir le mélange de l'étape (iv); la durée de l'étape (v) étant de 48 heures à 240 heures, grâce à quoi un produit brut ayant un rendement à la HPLC d'au moins 80% de 2,2'-oxodisuccinate est assuré;

(vi) on dilue le produit de l'étape (v) avec de l'eau et on en fait précipiter du carbonate de calcium; en arrêtant ainsi la réaction de l'étape (iv) et en abaissant ainsi le taux de calcium;

à condition que dans les étapes (iii) et (iv) simultanément:

- la durée totale à une température quelconque au-dessus de 100°C ne dépasse 15 minutes;
- la durée totale à une température quelconque au-dessus de 90°C ne dépasse pas 30 minutes;
- la durée totale à une température quelconque au-dessus de 80°C ne dépasse 2 heures;
- la durée totale à une température quelconque au-dessus de 70°C ne dépasse pas 6 heures;
- la durée totale à une température quelconque au-dessus de 65°C ne dépasse pas 12 heures; et

à condition que pour chaque mole d'acide malique préformé réagissant, la quantité molaire totale de réactif de type maléate, est de 1,1 à 1,6 mole; la quantité molaire totale de réactif de type calcium, est de 0,9 à 1,65 mole; la quantité molaire totale d'hydroxyde de sodium, est de 0,92 à 3,7 moles; l'excès net d'hydroxyde dans l'étape (iii), $M_{OH}$, est de 0,02 à 0,3 mole; et à condition en outre que pour chaque mole d'acide malique préformé mis en réaction, la quantité totale nette d'eau ajoutée dans les étapes (i), (ii) et (iii) ensemble, compensant les pertes par évaporation, n'est pas inférieure à 189 grammes et pas supérieure à 282 grammes.

11. Procédé selon la revendication 10 comprenant les étapes supplémentaires consistant (vii) à filtrer le mélange de l'étape (vi) pour retenir un gâteau de filtre et (viii) à utiliser le gâteau de filtre de l'étape (vii) comme source recyclée de carbonate de calcium dans l'étape (i).

12. Procédé selon l'une quelconque des revendications 1 à 11, dans lequel le malate préformé est fabriqué par un procédé comprenant au moins une étape de maintien, dans un réacteur, de préférence de structure étanche et ayant une pression dans l'intervalle de 0,21 MPa à 1,03 MPa, à une température de 120°C ou au-dessus, de manière encore préférée de 130°C à 220°C, pour une durée d'au moins 10 minutes, de manière encore préférée de 30 minutes à 6 heures, d'un mélange réactionnel aqueux comprenant:

I du maléate, ou des mélanges de ce dernier avec un ou plusieurs des composes: 2,2'-oxodisuccinate, fumarate et malate;

II des cations divalents choisis parmi le calcium, le magnésium et leurs mélanges; et

III un excès d'hydroxyde suffisant pour rendre alcalin ledit mélange réactionnel;

grâce à quoi, on est assuré d'obtenir ledit malate en des rendements atteignant et dépassant 90%.

13. Procédé selon la revendication 12 dans lequel le composant I est utilisé sous la forme d'anhydride maléique ou d'acide maléique et les composants II et III sont fournis simultanément sous la forme d'hydroxyde de calcium.

14. Procédé selon la revendication 12 ou 13, utilisant un rapport molaire calcium/maléate de 1,3:1 à 1,7:1 et une teneur en eau dudit mélange réactionnel dans l'intervalle de 45% à 73% en poids.

15. Procédé alcalin aqueux selon la revendication 12, pour obtenir le malate sous la forme d'un sel alcalin solide de calcium ou de calcium/magnésium mélangé par les étapes séquentielles consistant:

i. à faire réagir de l'anhydride maléique ou de l'acide maléique et du carbonate de calcium pour former un mélange;

ii. à permettre au dioxyde de carbone de se dégager;

iii. à rendre ledit mélange alcalin en utilisant de l'hydroxyde de calcium ou un mélange de ce dernier avec de l'hydroxyde de magnésium; à la condition d'ensemble que lesdits réactifs soient utilisés avec un rapport molaire (calcium + magnésium):maléate dans l'intervalle allant de plus de 1,3:1 à moins de 1,7:1, avec un rapport molaire calcium:magnésium dans l'intervalle de 1,00:0,00 à 0,80:0,20, avec une teneur en eau dans l'intervalle de 45% à 73% et un excès d'hydroxyde de 0,6 mole à 1,4 mole par mole de maléate utilisé; et

iv. à faire réagir ledit mélange dans un réacteur étanche à des températures dans l'intervalle de 140°C à 190°C pendant une période de 0,5 à 6 heures.